## Europäisches Patentamt

## European Patent Office

## Office européen des brevets

(11) Numéro de publication : **0 074 986**
**B1**

(12) **FASCICULE DE BREVET EUROPÉEN**

(45) Date de publication du fascicule du brevet :
18.09.85

(21) Numéro de dépôt : **82900965.3**

(22) Date de dépôt : **30.03.82**

(86) Numéro de dépôt international :
**PCT/FR 82/00060**

(87) Numéro de publication internationale :
**WO/8203330 (14.10.82 Gazette 82/25)**

(51) Int. Cl.⁴ : **A 61 K 39/29, G 01 N 33/576**

(54) **PROCEDE DE PREPARATION D'ANTIGENES DE L'HEPATITE VIRALE NANB, ET APPLICATION A LA REALISATION D'UN REACTIF PERMETTANT LE DIAGNOSTIC ET LE PRONOSTIC DES INFECTIONS PROVOQUEES PAR LES VIRUS DES HEPATITES VIRALES.**

(30) Priorité : **30.03.81 FR 8106385**

(43) Date de publication de la demande :
**30.03.83 Bulletin 83/13**

(45) Mention de la délivrance du brevet :
**18.09.85 Bulletin 85/38**

(84) Etats contractants désignés :
**AT BE CH DE FR GB LI LU NL SE**

(56) Documents cités :
**EP-A- 0 066 296**
**EP-A- 0 068 465**
**WO-A-80 /025 98**
**GB-A- 1 105 178**
**Le dossier contient des informations techniques présentées postérieurement au dépôt de la demande et ne figurant pas dans le présent fascicule.**

(73) Titulaire : **Trepo, Christian**
**22 Bis, rue des Essarts**
**F-69500 Bron (FR)**

(72) Inventeur : **Trepo, Christian**
**22 Bis, rue des Essarts**
**F-69500 Bron (FR)**

(74) Mandataire : **Nony, Michel et al**
**29, rue Cambacérès**
**F-75008 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**0 074 986**

## Description

La présente invention a pour objet un procédé de préparation d'antigènes de l'hépatite virale NANB, et l'application de ces antigènes à la réalisation d'un réactif permettant le diagnostic et le pronostic des infections, symptomatiques ou non, provoquées par les virus des hépatites virales.

Dans ce qui suit, on utilisera les abréviations suivantes :
— Ag signifie : antigène
— Ac signifie : anticorps
— HB signifie : hépatite B
— VHB signifie : virus de l'hépatite B
— NANB signifie : non-A non-B
— IgM signifie : immuno-globulines M
— IgG signifie : immuno-globulines G
— ID signifie : immunodiffusion
— CEP signifie : contre-électrophorèse
— DRI signifie : dosage radio-immunologique
— ELISA désigne un test immunoenzymatique (Enzyme linked immunosorbent assay)
— WH : hépatite de la marmotte.

Trois système Ag—Ac ont été découverts en relation avec le virus de l'hépatite NANB.

On sait que deux virus responsables de l'hépatite virale humaine ont été identifiés, le virus A et le virus B.

L'hépatite à virus A, volontiers épidémique, d'incubation courte (environ 30 jours) est transmise par voie « fécale-orale » et pratiquement jamais par transfusion.

L'hépatite due au virus B au contraire est observée volontiers mais non exclusivement chez les patients ayant subi des transfusions sanguines.

D'autres hépatites qui sont également précisément mais non exclusivement associées à la transfusion sanguine, ce qui illustre bien leur nature virale transmissible, ne sont dues ni au virus A ni au virus B (voir par exemple S. M. FEINSTONE et al., The New England Journal of Medicine, 10 Avril 1975, pages 707-710). Dans tous les pays, y compris la France, ces hépatites sont fréquentes et souvent asymptomatiques.

Leur incubation est variable, de 2 à 20 semaines, avec une fréquence maximale de 6 à 14 semaines. Leur aspect clinique ne permet pas de les distinguer des autres hépatites virales, toxiques ou médicamenteuses. Leur tendance à évoluer vers la chronicité est redoutable. Leur épidémiologie est voisine de celle des hépatites B. On les désigne par le terme « hépatite non-A non-B » (ou NANB).

Il existe une infectiosité prolongée du sang traduisant un partage chronique du virus avec une virémie persistante qui peut être complètement latente. 3 % au moins des donneurs de sang bénévoles et jusqu'à 30 % des donneurs payés aux U.S.A. paraissent ainsi infectés à leur insu et sont susceptibles de transmettre l'hépatite par la transfusion.

Malgré l'importance de ce problème on ne disposait jusqu'à présent d'aucune méthode, en dehors de la transmission au chimpanzé permettant de diagnostiquer qu'une hépatite était due à un virus non-A non-B ou qu'un donneur de sang pouvait être infectieux et transmettre la maladie au receveur de son sang ou des produits qui en sont dérivés. Les tests de dysfonctionnement hépatique les plus sensibles tels que l'élévation des transaminases SGPT ne sont susceptibles de reconnaître que 50 % de ces donneurs de sang infectieux. De plus le test des transaminases n'est pas spécifique car leur taux élevé est observé dans d'autres circonstances pathologiques hépatiques (lithiase biliaire, alcoolisme, etc.) n'ayant rien à voir avec l'hépatite virale NANB.

En 1978 SHIRACHI décrivit pour la première fois la détection en ID d'un Ag sérique présumé associé aux hépatites NANB. Le test en ID décrit ne paraissait cependant pas strictement spécifique des hépatites NANB puisqu'il se retrouvait positif dans 10 % des hépatites B. Au cours du symposium international, tenu en Juin 1980 à Vienne, consacré aux hépatites NANB plusieurs tests d'ID décrits par divers auteurs y compris SHIRACHI se sont avérés non reproductibles et non spécifiques puisqu'ils ont conduit à considérer comme positif un sérum témoin (voir aussi SUH, Lancet 1:178-180, 1981). Le réactif de l'invention permet d'éviter ces inconvénients.

Les tests ou dosages radio-immunologique décrits par d'autres auteurs se sont également avérés non spécifiques ; voir NEURATH, Journal Gen. Virol., 48 285-295 (1980). On va décrire ci-après les travaux qui ont conduit à la mise au point du réactif de l'invention.

En faisant réagir des sérums de polytransfusés ou de convalescents d'hépatite NANB avec des sérums prélevés à la phase précoce de la maladie, il a été possible de mettre en évidence, grâce à l'immuno-diffusion, des lignes de précipitation entre des sérums de convalenscents ou de polytransfusés, et les sérums d'hépatite NANB à la phase aiguë. Ces lignes de précipitation identifiant des Ag nouveaux spécifiques des hépatites NANB ont servi de marqueurs pour l'identification du virus en cause.

Les résultats de ces travaux ont permis d'identifier ainsi un virus responsable d'hépatites NANB présentant une similitude morphologique avec le virus de l'hépatite B, y compris des réactions sérologiques croisées.

Les mêmes travaux ont permis d'identifier 2 systèmes Ag/Ac associés à ce virus désignés antigène

2

NANB/e et NANB/c par analogie avec les antigènes homologues de l'hépatite B ; voir C. TREPO et al., C.R. Acad. Sc. Paris, t. 290, pages 343-346 (1980).

L'Ag NANB/e réagissait en identité en ID avec un Ag présent dans le sérum de référence de SHIRACHI et est donc supposé analogue à cet Ag désigné par cet auteur HC comme nous y reviendrons. Un Ag NANB/s caractéristique de l'enveloppe du virus a été également mis en évidence.

On sait que les hépatites virales ou non sont accompagnées d'une augmentation caractéristique du taux des transaminases TGO (transaminases glutamique oxalacétique) et TGP (transaminases glutamique pyruvique).

Cette augmentation plus ou moins importante des transaminases est le test admis par les spécialistes pour diagnostiquer les hépatites, qu'elles soient virales ou non.

On ne considère comme hépatite virale NANB que les cas où l'on peut éliminer le rôle d'agents toxiques et médicamenteux ainsi que celui des virus HB et HA en constatant l'absence des antigènes, et/ou d'anticorps signant la présence de ces virus, selon les méthodes connues décrites par exemple dans les publications suivantes :

— Organisation Mondiale de la Santé, Progrès en matière d'hépatite virale, Sér. Rapp. Techn., 1977, n° 602, 1-68 ; A. J. Zuckermann, The Three types of human viral hepatitis, Bull. O.M.S., 1977, *56*, 1-20 ; R. SOHIER, Diagnostic des maladies à virus, Flammarion, Medecine Sciences Edition, Paris, 1964, et mises à jour jusqu'à 1979.

En outre, on a vérifié que le virus EPSTEIN-BARR et le virus cytomégalique n'étaient pas en cause. Ces critères représentent la définition des hépatites NANB, qui se fait donc, en l'absence de test diagnostic spécifique disponible actuellement par exclusion des autres causes.

On peut donc selon ces critères sélectionner le sérum des sujets, symptomatiques ou non, soupçonnés d'être atteints d'hépatite NANB.

En procédant à des essais d'immuno-diffusion en milieu approprié entre les sérums ou entre ces sérums et des homogénéisats de foie, on a observé des réactions de précipitation de complexes antigène-anticorps. Parfois, plus d'une ligne de précipitation étaient observées. La comparaison avec un échantillon authentique de l'antigène décrit par SHIRACHI a permis d'établir que la ligne de précipitation la plus fréquente en ID initialement désignée Ag NANB (Vitvitski et al., 1979 Lancet 2:1263-1267) présentait une réaction d'identité avec un Ag présent dans l'échantillon de SHIRACHI. L'Ac correspondant se trouvait présent dans le réactif. D'autres molécules réactives parasites semblaient cependant présentes dans cet échantillon Ag et Ac, comme c'est souvent le cas, ce qui explique la non spécificité des résultats de ces auteurs présentés au symposium de Vienne.

Par ailleurs l'Ag NANB décrit est apparu avoir une réactivité croisée avec le système HBe, et plus précisément avec Ag HBe/3 et non avec les autres spécificités HBe/1 et 2 (Vitvitski et al., J. Virol. Methods, 1:149-156, 1980).

Cette démonstration faisait supposer que l'Ag NANB décrit était l'équivalent pour le virus NANB de l'Ag HBe/3. Les propriétés physico chimiques de l'Ag NANB (HANTZ et al., J. Med. Virology 5:73-86 (1980) et sa localisation dans le noyau des hépatocytes renforçaient cette observation.

L'Ag NANB a donc été redésigné NANBe, et on suppose que l'Ag décrit par SHIRACHI correspond à cet Ag NANBe, compte tenu de la réaction observée.

En vue de l'identification d'autres systèmes Ag/Ac spécifiques les couples de sérums donnant une double ligne de précipitation ont été sélectionnés. Une ligne de précipitation additionnelle, distincte de celle de l'Ag NANB/e, se trouvait reproductiblement située, par rapport à celle-ci, du côté du puits où on avait déposé le sérum à tester contenant l'antigène, la ligne de l'Ag NANB/e se trouvant, elle, plus près du puits contenant le réactif anticorps.

L'ultracentrifugation des sérums ainsi sélectionnés a permis de retrouver dans les culots de centrifugation, l'activité antigénique responsable de cette deuxième ligne de précipitation alors que l'activité antigénique NANB/e n'est pas retrouvée dans les culots après ultracentrifugation et reste dans le surnageant.

Le nouvel antigène ainsi isolé a permis ensuite de sélectionner, par réaction d'immuno-diffusion par exemple, des sérums contenant l'anticorps correspondant.

On sait que depuis l'examen systématique des sérums de donneurs de sang, dans la plupart des pays, pour la présence d'antigènes du virus de l'hépatite B, plus de 80 % des hépatites post-transfusionnelles observées, sont des hépatites NANB.

Généralement, le nouvel antigène découvert, appelé ci-après Ag NANBs, est détecté dans les sérums de 10 à 20 % environ des patients souffrant d'hépatite post-transfusionnelle, ainsi que chez des sujets asymptomatiques présentant une élévation de transaminases, TGP surtout, supérieure ou égale à deux fois la valeur normale.

Les anticorps anti-NANBs (ou Ac anti-NANBs) sont détectés en proportion importante, chez les convalescents, généralement 1 à 12 mois après normalisation du taux des transaminases. Ils sont également très fréquents chez les sujets polytransfusés ou exposés de par leur activité à des contacts répétés avec le virus.

La suite des essais décrits ci-dessus permet de détecter et d'isoler l'Ag NANBs et l'anticorps correspondant, même si on ne dispose pas d'un échantillon de référence. Une série de travaux permis de démontrer que l'Ag NANBs est un Ag d'enveloppe du virus NANB.

Des recherches supplémentaires ont encore permis la découverte d'un autre antigène, associé à la nucléocapside du virus NANB, désigné Ag NANBc (antigène de « core »), qui sera décrit davantage ci-après.

L'intérêt propre des 3 systèmes Ag—Ac définis ci-dessus pour la prévention, le pronostic, le diagnostic et le traitement des hépatites NANB est variable, les 3 réactions se complétant.

Ainsi, il existe pour le virus NANB comme pour le VHB 3 systèmes Ag/Ac distincts associés au virus :

1) Un Ag de surface ou Ag NANBs forme donc l'enveloppe du virus complet et se trouve synthétisé également en excès sous forme de petites particules très variables en taille dans le sérum. Lors de l'infection, cet Ag est produit par le cytoplasme des hépatocytes infectés et également exprimé sur la membrane de la cellule.

Les Ac anti-NANBs sont les Ac qui agglutinent ces particules virales synthétisées en excès ainsi que le virus complet. Ce sont donc les Ac protecteurs. Leur présence chez un individu le protège contre l'infection comme cela a été montré par l'administration des gammaglobulines de convalencents.

Les Ag NANBs peuvent servir de marqueur de l'infection et permettre de faire le diagnostic d'une virémie NANB. Néanmoins la présence fréquente d'Ag en quantité faible, ou l'association de cet Ag et d'Ac sous forme d'immuns complexes explique que, bien souvent, il soit difficile de mettre en évidence par des techniques peu sensibles l'Ag NANBs. La réalisation d'un test radioimmunologique ou immunoenzymatique ou d'un test d'hémagglutination permet grâce au gain de sensibilité de détecter plus facilement les porteurs de virus par mise en évidence de faibles quantités d'Ag NANBs.

2) Le système antigénique NANBc correspond à la nucléocapside du virus NANB complet qui, dans le sérum, est enveloppé. L'Ag NANBc n'est donc pas libre dans le sérum. On ne le révèle qu'après purification des virions et éclatement de l'enveloppe par des détergents doux (par exemple ceux désignés par les marques commerciales Tween 80 ou Triton X100). L'Ag NANBc est synthétisé dans le noyau des hépatocytes infectés où il est facilement mis en évidence par immunofluorescence et également en microscopie électronique, où on peut détecter des particules caractéristiques qui ont l'aspect ultrastructural des nucléocapsides en voie de formation dans les noyaux. L'absence d'Ag NANBc libre dans le sérum (puisque les nucléocapsides sont enveloppées) ainsi que la multiplication des capsides NANBc dans le noyau des cellules infectées et leur libération lors de la destruction de celles-ci, réalisant une stimulation permanente, aboutit à la présence dans le sérum d'anticorps anti-NANBc dès le début de l'infection : ces anticorps anti-NANBc qui sont détectables par immunofluorescence indirecte mais aussi par d'autres tests (contreélectrophorèse ou dosage radioimmunologique ou ELISA), témoignent d'une infection en cours d'évolution. Si les anticorps anti-NANBc contiennent des anticorps de la classe IgM, il s'agit d'une infection en cours ou qui date de moins de 8 semaines, donc qui est toujours actuelle. S'il n'y a que des anticorps anti-NANBc de la classe IgG, ceci traduit une infection passée, avec arrêt de la multiplication du virus depuis un temps variable.

L'intérêt essentiel du système Ag/Ac NANBc est donc le diagnostic des infections à virus NANB soit aiguës soit chroniques et la possibilité de distinguer les infections actuelles des infections passées (selon la présence ou l'absence d'IgM).

Il faut insister sur l'intérêt de ce système pour faire un réactif à visée diagnostique. En effet dans les infections à virus NANB les Ag NANBe et NANBs qui servent également au diagnostic peuvent être masqués sous forme d'immuns complexes à certains moments de l'évolution. Dans ces conditions, seul l'anticorps anti-NANBc permettra de préciser qu'un sujet est infecté et/ou infectieux. Ce système sera très important pour le diagnostic des porteurs de virus en particulier chez les donneurs de sang afin de reconnaître les donneurs infectieux et exclure leur sang de la transfusion.

On a également démontré qu'il existe des réactivités croisées entre les Ag NANBc des diverses souches de virus NANB obtenus à partir de foies infectés par ces différentes souches. Il est donc intéressant d'utiliser pour le réactif de l'invention plusieurs antigènes NANBc de ces différentes souches afin d'avoir le test le plus polyvalent possible en particulier pour la détection des donneurs de sang dangereux, ces divers antigènes NANBc étant de préférence fixés sur (ou utilisés avec) une même unité de réactif. De même on peut utiliser pour un même réactif à la fois un Ag NANBc et un Ag HBc.

Il a également été découvert qu'il y avait une faible réactivité croisée entre les Ag NANBc et HBc. Dans certains cas, un sérum faiblement positif en dosage radio-immunologique pour l'Ac anti-HBc révèle en fait une infection à virus NANB et vice versa.

3) Le troisième système d'Ag et d'Ac lié au virus NANB est le système Ag/Ac NANBe.

Ce système est celui qui a été découvert initialement et appelé d'abord Ag NANB avant d'être redésigné Ag NANBe.

Il serait susceptible de correspondre au système décrit par SHIRACHI. Ce système antigénique a un intérêt diagnostique important car la détection de l'Ag NANBe ou de l'Ac correspondant permet d'identifier des porteurs de virus ou de reconnaître que le virus NANB est responsable d'une hépatite aiguë ou chronique.

Les recherches du déposant ont montré que tant les sujets porteurs de l'Ag NANBe que ceux porteurs de l'Ac anti-NANBe semblent activement infectés par le virus, comme en témoignent les tests d'immunofluorescence et la microscopie électronique effectués sur des biopsies hépatiques.

Un des intérêts de l'Ag NANBe est qu'il apparaît être un Ag de groupe commun à plusieurs virus de la même famille. L'Ag NANBe paraît être l'Ag pour lequel il existe la réactivité croisée la plus importante entre les divers virus de cette famille. Aussi, entre l'Ag e3 du VHB, l'Ag NANBe et aussi l'Ag e du virus de l'hépatite de la marmotte, la réactivité croisée est considérable. Cet Ag/e se retrouve également chez d'autres espèces comme l'écureuil souterrain.

L'intérêt essentiel de cet Ag-e est donc qu'il apparaît être commun à toute une série de virus hépatitiques de l'homme et des animaux, formant une nouvelle famille de virus que certains suggèrent de désigner HEPA-DNA virus. Cela explique également que des infections par des souches de virus NANB distinctes du point de vue de leur immunité, en particulier chez le chimpanzé donnent une réaction positive pour l'Ag NANBe. Ceci confère donc à cet antigène une valeur diagnostique importante et complémentaire de celle des anti-NANBc.

Une autre caractéristique importante de l'Ag NANBe est d'être synthétisé dans les infections NANB, en grand excès, souvent à un titre supérieur à l'Ag NANBs, ce qui permet un gain de sensibilité.

Comme indiqué précédemment, il existe une réactivité croisée d'intensité croissante lorsqu'on passe des Ag les plus superficiels ou de surface des virus de l'hépatite aux Ag de nucléocapside core ou « e ».

Ainsi les Ag NANBs et HBs n'ont que très peu de réactivité croisée exclusivement démontrable par des tests très sensibles, alors que les Ag NANBc et NANBe ont une réactivité croisée plus importante avec les Ag HBc et HBe/3 respectivement.

Compte tenu du mode de réalisation pratique des tests commerciaux des Ag du VHB actuellement sur le marché la réactivité croisée n'est pas utilisable pour le diagnostic des hépatites NANB.

Les 3 systèmes Ag/Ac décrits ci-dessus peuvent servir de base à la réalisation de tests diagnostiques pour les sujets infectés par le virus NANB, qu'ils soient symptomatiques ou non. Plusieurs types de réactifs ont été réalisés. Leurs intérêts sont distincts et souvent complémentaires.

Il convient de noter que dans le cas des infections NANB, les Ac naturels obtenus dans le sérum des malades sont insuffismment spécifiques et ne peuvent pas être utilisés tels quels pour construire des tests. Les Ac dont on a besoin pour les réactifs de l'invention et leur utilisation ne peuvent être obtenus qu'après purification des Ag par exemple selon les procédés qui seront décrits ci-après.

La présente invention a notamment pour objet un procédé de préparation d'antigènes purifiés de l'hépatite virale NANB, caractérisée par le fait que l'on sélectionne des sérums ou des extraits de foie dans lesquels on a reconnu la présence de virus NANB, que l'on procède à une ultracentrifugation pour concentrer lesdits virus dans le culot de centrifugation, que l'on recueille ledit culot et le traite par un détergent non ionique, que l'on laisse incuber pendant 1 à 24 heures environ à température comprise entre 0 et 37 °C, que l'on procède à un fractionnement et repère les fractions contenant l'Ag NANBc purifié, selon les techniques connues, notamment les techniques basées sur la réaction antigène-anticorps, isole les fractions contenant de l'Ag NANBc purifié, puis que, si désiré, on soumet lesdites fractions à l'action d'un détergent ionique, laisse incuber pendant 1 heure à 24 heures environ à température de 0 à 37 °C, et isole l'Ag NANBe.

Pour détecter la présence de virus NANB, on utilise par exemple les méthodes mentionnées dans l'article de C.R. Ac. Sc. Paris, t-289 (17 décembre 1979) pages 1263-1266, et notamment le repérage d'une activité ADN polymérase.

Parmi les détergents non ioniques on citera, notamment les aryl polyéthers de glycol alkylés tels que le Triton X100, les polyoxyéthylène sorbitan monooléates tels que le Tween 80, ou encore le NP 40.

Parmi les détergents ioniques on citera notamment les détergents anioniques tels que le SDS (dodécyl sulfate de sodium).

Pour purifier l'Ag NANBe, selon un procédé également objet de l'invention, on peut aussi opérer comme suit : on sélectionne des sérums positifs pour l'Ag NANBe, on élimine les particules virales éventuellement présentes, par exemple par ultracentrifugation, on élimine les gammaglobulines selon les méthodes connues, par exemple par précipitation au sulfate d'ammonium, on concentre les fractions contenant l'Ag NANBe, notamment par précipitation, par exemple à l'aide de sulfate d'ammonium ou de polyéthylèneglycol, puis on chromatographie les fractions concentrées contenant l'Ag NANBe sur un support sur lequel est fixée de l'héparine, on élue avec une solution aqueuse de chlorure de sodium de concentration croissante, recueille les éluats contenant l'Ag NANBe, et élimine les sels selon les méthodes usuelles. Il convient de noter que ce procédé est également utilisable pour purifier l'Ag NANBs, en sélectionnant comme produits de départ des sérums positifs pour l'Ag NANBs. On peut chromatographier par exemple sur gel d'agarose sur lequel est fixée de l'héparine (héparinogel, marque commerciale) et recueillir les éluats correspondant à une concentration en ClNa de 0,3 à 0,7 M. On élimine les sels (principalement ClNa) par exemple par dialyse.

L'invention a également pour objet l'obtention d'antigènes purifiés NANB, mettant à profit l'antigénicité croisée notable entre les virus NANB et HB. Ce procédé est caractérisé par le fait que l'on hyperimmunise des animaux avec une fraction purifiée en un antigène de l'hépatite B, que l'on utilise les gammaglobulines produites par les animaux hyperimmunisés pour obtenir les lignes de précipitation avec des sérums contenant un antigène NANB correspondant et exempts d'antigènes HB, et que l'on utilise les gammaglobulines ayant donné des lignes de précipitation pour réaliser des supports de chromatographie d'affinité qui permettront de fixer et purifier les antigènes NANB.

**0 074 986**

Dans le cas où on a hyperimmunisé les animaux avec Ag HBc ou Ag HBe3, les anticorps obtenus sont directement utilisables en chromatographie d'affinité pour fixer les antigènes NANB.

Dans le cas où l'on cherche à obtenir des anticorps donnant une ligne de précipitation avec l'Ag NANBs, il est particulièrement utile d'hyperimmuniser les animaux avec de l'Ag HBs possédant le déterminant a, par exemple l'Ag HBs de sous-type Ad et/ou ay. On peut notamment hyperimmuniser les animaux à l'aide de Ag HBs ad, puis de Ag HBs ay (ou vice versa), afin d'augmenter la proportion d'anticorps contre le déterminant a. On utilise les gammaglobulines des animaux ainsi hyperimmunisés pour obtenir des lignes de précipitation avec des Ag NANBs. On peut alors recueillir les lignes de précipitation obtenues et les administrer directement, après mélange avec un adjuvant, à des animaux du même genre, qui peuvent être soit des animaux neufs, soit les animaux ayant produit les anticorps donnant une ligne de précipitation avec les AG NANBs.

De même on peut procéder à une nouvelle hyperimmunisation avec les lignes de précipitation obtenues de façon analogue avec l'Ag NANBe ou NANBc.

La présente invention a également pour objet un réactif permettant le diagnostic de l'hépatite virale NANB ou permettant de révéler le stade d'évolution de la maladie, y compris la guérison, caractérisé par le fait qu'il comprend un support (ou substrat) solide sur lequel est fixé au moins un antigène NANBc, NANBe ou NANBs et/ou au moins un anticorps anti-NANBc, anti-NANBe ou anti-NANBs.

L'antigène ou l'anticorps peut être fixé sur le support solide par tout moyen connu pour la fixation des antigènes et des anticorps, par exemple par adsorption, par liaison covalente ou non, par exemple par couplage chimique avec un agent de couplage bifonctionnel ou par affinité immunochimique.

Le support peut être réalisé avec tout matériau solide biologique (globules rouges) ou synthétique doué de propriétés adsorbantes ou capable de fixer un agent de couplage. Ces matériaux sont connus et décrits dans la littérature. Parmi les matériaux solides capables de fixer les Ag ou Ac par adsorption on citera par exemple le polystyrène, le polypropylène ou les latex. Parmi les matériaux utilisables pour fixer les Ag ou les Ac par covalence à l'aide d'un agent de couplage, on cite notamment le dextran, la cellulose, leurs dérivés aminés tels que diéthylamino-cellulose ou diéthylamino-dextran.

Le support se présente par exemple sous forme de disque, de tube, de baguette ou de bille.

Les agents de couplage permettant de fixer par covalence les Ag ou les Ac sur le support solide sont connus ; ce sont par exemple des dérivés carboxyliques, des dérivés biofonctionnels tels que les dialdéhydes (glutaraldéhyde), des diisocyanates (toluène-diisocyanate), des quinones (benzoquinone). Les Ac ou les Ag peuvent également être fixés sur des supports solides selon les méthodes décrites par exemple dans les brevets français FR-B-2 319 399, FR-B-2 403 556 et FR-B-2 403 098.

Selon un mode de réalisation préféré, on utilise comme support des billes ou des tubes de polystyrène. De telles billes ayant par exemple 1 à 5 mm de diamètre, peuvent être obtenues dans le commerce, comme cela est indiqué dans la partie expérimentale ci-après.

Selon un premier mode de réalisation, le réactif de l'invention contient de l'anticorps spécifique fixé sur le support solide.

L'anticorps est un anticorps spécifique de l'Ag à détecter (anti-NANB/c, /e ou /s).

Ce réactif permet notamment de détecter la présence de l'antigène correspondant, selon la méthode dite « sandwich », par mise en contact avec un sérum à tester. Après lavage pour éliminer les molécules non fixées puis mise en contact ultérieure avec une préparation du même anticorps couplé à un agent traceur (encore appelé anticorps conjugué), on lave pour éliminer l'excès d'anticorps conjugué non fixé puis on examine si l'anticorps couplé au traceur s'est fixé ou non sur le support. C'est l'étape dite de révélation. Il est ainsi possible de déterminer si le sérum testé contenait l'antigène à détecter. En effet, si l'anticorps couplé à l'agent traceur se trouve fixé sur le support c'est que sur le support s'est formé le complexe anticorps-antigène-anticorps conjugué, et que l'antigène recherché était donc présent. Dans le cas contraire, on peut conclure à l'absence de l'antigène recherché dans le sérum testé.

Ce réactif permet également la détection des Ag correspondants selon une méthode dite de « compétition ». Pour cela, on met en contact le réactif avec le sérum à tester. Après lavage pour éliminer les molécules non fixées, on met en contact le réactif avec une préparation d'Ag couplé à un traceur, l'Ag étant celui contre lequel est dirigé l'Ac fixé au support. On lave à nouveau puis on examine le réactif pour rechercher la présence ou l'absence de l'antigène conjugué. Si l'Ag conjugué s'est fixé sur le réactif, c'est que les sites anticorps étaient libres et que le sérum à tester ne contenait donc pas d'Ag contre l'Ac fixé au support. Si l'Ag conjugué ne se fixe pas ou ne se fixe qu'en faible proportion, par exemple si la quantité fixée est inférieure de plus de 50 % à la quantité fixée dans le cas d'un sérum témoin ne contenant pas l'Ag recherché, c'est que le sérum étudié contenait le même Ag que celui couplé au traceur, et que le complexe Ac−Ag formé a masqué les sites anticorps et a empêché la fixation de l'Ag conjugué sur l'Ac fixé au support.

Selon un deuxième mode de réalisation, le réactif de l'invention contient de l'antigène fixé sur le support solide. L'antigène fixé au support est l'Ag NANB/c, /e, ou /s.

Ce réactif permet de détecter la présence des anticorps correspondant à l'antigène fixé selon le principe de la méthode « sandwich » déjà décrite ci-dessus, en remplaçant bien entendu l'anticorps conjugué par un Ag couplé à un traceur, cet Ag étant celui contre lequel est dirigé l'Ac recherché. Selon que l'Ag conjugué s'est fixé ou non, on conclura à la présence ou à l'absence de l'Ac recherché.

De même, le réactif de ce deuxième mode de réalisation peut servir à détecter la présence des anticorps anti-NANBs, anti-NANBc ou anti-NANBe selon la méthode de compétition déjà décrite, le conjugué étant cette fois l'anticorps correspondant couplé à un traceur. Selon que l'Ac conjugué se fixe ou ne se fixe qu'en faible proportion, on conclura à l'absence ou à la présence de l'Ac recherché.

Il convient d'insister sur le fait que les anticorps utilisés pour la réalisation ou l'application des réactifs de l'invention doivent être des préparations d'anticorps spécifiques obtenus soit par hyperimmunisation selon les méthodes connues à l'aide d'antigènes hautement purifiés soit par chromatographie d'affinité, soit par production d'Ac monoclonaux. On a en effet constaté que, contrairement à ce qui était observé pour l'HVB, les anticorps naturels prélevés chez l'homme ne permettaient pas d'obtenir un réactif suffisamment sensible.

Bien entendu, les divers Ag utilisés pour la réalisation de l'application des réactifs de l'invention sont nécessairement des Ag hautement purifiés.

Selon un mode de réalisation particulier, l'Ag NANB du réactif destiné à la recherche de l'anticorps correspondant peut être fixé sur le support par l'intermédiaire des anticorps du sérum à tester, selon une méthode« sandwich inverse ». Dans ce cas on utilise à titre de produit intermédiaire le réactif constitué par de l'anticorps anti-(gammaglobulines M ou G humaines) fixé sur le support. Ce réactif intermédiaire, mis en contact avec le sérum à tester, fixe les gammaglobulines M ou G sérum.

Pour utiliser le réactif ainsi obtenu, on met en contact le support, revêtu de gammaglobulines M ou G du sérum à tester par l'intermédiaire des anti-gammaglobulines M ou G humaines, avec une préparation purifiée d'antigène de l'hépatite NANB, par exemple une préparation purifiée d'Ag NANBc. On met ensuite en contact le support avec une préparation d'anticorps correspondant audit antigène purifié, ledit anticorps étant couplé à un traceur. on observe ensuite par révélation selon les méthodes usuelles si l'anticorps couplé au traceur s'est fixé sur le support. S'il est fixé, c'est que le sérum étudié contenait des anticorps contre ledit antigène purifié, et s'il n'est pas fixé, le sérum étudié ne contenait pas ledit anticorps. Il est en particulier intéressant d'utiliser le support intermédiaire décrit ci-dessus contenant des anticorps contre les différentes classes M ou G d'immunoglobulines anti-IgM humaines ou anti-IgG humaines, ce qui permet de distinguer les différents stades d'évolution de la maladie, comme cela a été indiqué ci-dessus. On peut également rechercher les anticorps NANB du sérum à tester par addition d'Ag correspondant couplé à un traceur. Si l'Ag conjugué se fixe, c'est que l'anticorps correspondant était présent dans le sérum.

De préférence, l'incubation du réactif avec le sérum à tester et avec les préparations d'Ag ou d'anticorps couplé au traceur est effectuée de préférence à pH alcalin (par exemple à pH de 7 à 9), en laissant incuber pendant 1 à 48 heures, à température comprise entre 5 et 50 °C environ.

Généralement la quantité d'antigène ou d'anticorps fixée sur une unité de réactif (par exemple une bille de polystyrène) est la suivante :

anticorps : de 1 à 20 µg, par exemple 10 µg environ ;

antigène : de 0,1 à 10 µg, par exemple 1 µg environ.

Le traceur est un traceur radioactif ou enzymatique.

Le traceur peut être un traceur radioactif obtenu par exemple par échange isotopique avec un isotope radioactif tel que I 125.

Le couplage des anticorps ou des antigènes avec un agent traceur radioactif est connu en soi et peut être réalisé par exemple selon la méthode décrite par GREENWOOD.

La radioactivité éventuellement présente sur le réactif de l'invention après réalisation du test est évaluée selon les méthodes usuelles à l'aide d'un compteur approprié (compteur gamma, ou à scintillation).

Le couplage des anticorps ou Ag avec un traceur enzymatique est connu en soi et peut être réalisé par exemple selon une méthode analogue à celle décrite par NAKANE, Journal Histochemistry Cytochemistry, 22 : 1984-1991 (1974) qui consiste à fixer l'enzyme sur des molécules d'IgG supports de la fonction Ac. L'enzyme est par exemple une peroxydase ou une phosphatase alcaline. L'activité enzymatique éventuellement présente sur le réactif après réalisation d'un test peut être déterminée selon les méthodes connues avec un substrat approprié permettant par exemple une révélation par colorimétrie, par fluorescence, par luminescence ou par potentiométrie.

I. Réactifs utilisant le système Ag NANBs.

1. Intérêts :

Cet Ag est spécifique de l'enveloppe du virus NANB. Le virus NANB plus fréquent est désigné par NANB1, et la description qui suit concerne plus particulièrement ce virus, pour simplifier. Il est bien entendu que l'antigène de surface de tout virus apparenté donnant une réaction d'immunité croisée avec le virus NANB1 est également utilisable.

Cet Ag de surface apparaît dans le sérum des sujets infectés par le virus NANB 2 à 4 semaines après une transfusion ou une piqûre accidentelle. Il précède l'élévation des transaminases quand elle se produit de 1 à 8 semaines parfois plus. Il persiste pendant toute la durée de leur élévation et souvent après qu'elles soient revenues à la normale. Sa présence est un signe de virémie NANB et donc d'infectivité.

Sa mise en évidence traduit donc la présence du virus NANB dans le foie et le sérum, et l'infectivité du sang de la personne porteuse qui est donc capable soit de transmettre une hépatite en donnant son sang, soit de la communiquer lors de ses contacts intimes.

Dans les hépatites chroniques, l'Ag NANBs est présent de façon persistante.

Cet Ag peut être masqué dans certaines conditions sous forme de complexes antigène-anticorps, et l'on peut détecter les anticorps anti-NANBs chez certains sujets malades. Leur apparition est signe d'amélioration clinique, mais peut être pas de façon immédiate signe de guérison.

Certains sujets peuvent être porteurs de complexes Ag/Ac NANBs/anti-NANBs en excès d'Ac, qui seuls sont alors détectés. Ces sujets sont toujours infectieux.

L'Administration d'Ag NANBs à des animaux y compris l'homme, provoque chez ceux-ci le développement d'une réponse anticorps. Une telle administration permet soit d'obtenir des anticorps correspondants, soit de réaliser une vaccination contre l'hépatite NANB.

2. Réalisation d'un dosage radioimmunologique en phase solide de type sandwich pour la détection de l'Ag NANBs.

a) purification de l'Ag NANBs :

On rappelle que l'Ag NANBs présente les caractéristiques suivantes :

— Densité : entre 1,20 et 1,30 g/ml en solution de chlorure de césium, et entre 1,15 et 1,25 g/ml en solution de saccharose ;

— migration électrophorétique dans la zone des $\alpha$-$\beta$-globulines ;

— il est associé en particulier à des particules d'allure virales dont les plus caractéristiques ont des tailles variant de 10 à 45 nm de diamètre, le virion complet apparaissant comme une sphère à double enveloppe de 35 à 45 nm ;

— lorsqu'il est administré à un animal ayant un système immunitaire, il provoque la formation d'anticorps avec lesquels il donne une réaction de précipitation, ledit anticorps étant en outre capable d'agréger et/ou de précipiter lesdites particules virales complètes à double enveloppe contenant une nucléocapside, ayant des dimensions de 35-45 nm environ, ledit anticorps étant également capable de provoquer, lorsqu'il est couplé avec un agent de fluorescence, une fluorescence cytoplasmique et/ou membranaire dans des coupes de tissu de foie provenant de sujets atteints d'hépatite NANB ; ledit anticorps n'étant capable ni d'agréger ou de précipiter les particules complètes du virus de l'hépatite B, ni de provoquer une fluorescence cytoplasmique et/ou membranaire dans des tissus de foie de sujets atteints d'hépatite B.

L'utilisation d'un échantillon d'anticorps anti-NANBs permet de sélectionner facilement les sérums contenant l'Ag NANBs, ceux-ci pouvant servir à leur tour à l'identification des sérums contenant l'Ac anti-NANBs.

Pour purifier l'Ag NANBs, on sélectionne des sérums ou des plasmas dans lesquels on a repéré, selon les méthodes classiques telles que les réactions d'immunodiffusion, de contre-électrophorèse, d'inhibition de l'immunofluorescence, ou de radio-immunologie, la présence d'antigène NANBs, et on purifie ledit antigène selon les méthodes classiques de purification des protéines.

Cette purification peut être effectuée en utilisant par exemple au moins une des méthodes suivantes :

— chromatographie d'affinité,

— ultracentrifugation, par exemple en gradient de saccharose ou de césium ;

— chromatographie sur gel, en particulier sur héparinogel ;

— précipitation fractionnée à l'aide d'un agent précipitant tel que les polyols, par exemple le polyéthylèneglycol, ou tel que le sulfate d'ammonium,

— ultrafiltration à l'aide d'une membrane dont la dimension des pores est telle qu'elle retient les molécules ayant un poids moléculaire supérieur à 30 000.

L'antigène NANBs étant parfois présent sous forme d'immunocomplexes, il est alors avantageux de dissocier lesdits complexes soit avant l'opération de purification soit en purifiant dans des conditions assurant cette dissociation (par exemple à pH suffisamment acide ou suffisamment alcalin).

Pour purifier l'antigène par chromatographie d'affinité, on peut par exemple procéder à une chromatographie sur un support, de préférence poreux, dont la surface est tapissée d'un revêtement de molécules anticorps anti-NANBs reliées au support par l'intermédiaire d'un agent de couplage. Le support est par exemple constitué par du Sépharose. L'agent de couplage est par exemple un halogénure de cyanogène. On dispose l'immunoabsorbant dans une colonne, on applique à cette colonne une solution de l'antigène à purifier, on lave avec un tampon. On élue ensuite l'antigène, un agent dissociant la liaison antigène-anticorps, par exemple avec une solution tampon à pH acide ou au contraire alcalin, car ces pH extrêmes entraînent une dissociation de la liaison entre Ag et Ac. On collecte des fractions dans lesquelles on repère la présence de protéines par mesure de la densité optique à 280 nm, et la présence de l'antigène NANBs est détectée dans les fractions, par les réactions sérologiques avec l'Ac anti-NANBs : CEP (contre-électrophorèse), et/ou inhibition de l'immunofluorescence et de la radioimmunoprécipitation.

Pour mettre en œuvre une purification par ultracentrifugation, on prépare un gradient d'une solution d'un agent tel que le sucrose ou le chlorure de césium, on dépose la solution à purifier sur ledit gradient et l'on procède à une ultracentrifugation. On recueille les fractions dans celles correspondant à une densité de 1,20 à 1,30 en CsCl et 1,15-1,25 en sucrose on retrouve en sérologie l'Ag NANBs.

# 0 074 986

Pour réaliser une purification par chromatographie sur gel, on dispose dans une colonne un gel de matériau hydrophile poreux, on applique sur la colonne une fraction contenant l'antigène à purifier et l'on élue l'antigène. On opère de préférence en milieu alcalin, qui a un effet dissociant et qui permet donc de récupérer l'Ag NANBs présent sous forme d'immunocomplexes.

On collecte des fractions dans lesquelles on repère la présence de protéines par mesure de la densité optique à 280 nm. L'antigène NANBs est détecté dans ces fractions, par les réactions sérologiques indiquées précédemment.

De préférence, pour mettre en œuvre toutes les méthodes de purification, on utilise comme produit de départ un sérum ou un plasma défibriné et concentré, par exemple de 2 à 10 fois. La concentration peut être réalisée par exemple par précipitation des protéines, notamment au polyéthylèneglycol ou au sulfate d'ammonium et redissolution dans une solution aqueuse tamponnée. On détermine de façon simple la quantité d'agent précipitant nécessaire, par exemple sulfate d'ammonium à 60 %.

Toutes les méthodes rappelées ci-dessus pour la purification de l'Ag NANBs peuvent être utilisées également pour la purification des Ag NANBc et NANBe, avec les adaptations nécessaires.

b) préparation d'anticorps anti-NANBs.

L'Ag purifié obtenu au paragraphe précédent sert à immuniser des animaux qui permettent d'obtenir des anticorps anti-NANBs spécifiques.

Pour cela on administre à un animal une préparation d'antigène NANBs purifiée telle que décrite ci-dessus en combinaison avec de l'adjuvant de FREUND complet ou avec tout autre adjuvant. On effectue au moins une administration et de préférence plusieurs. Le sang de l'animal est prélevé au moment du taux optimum des Ac voulus, au bout de 1 à 4 mois par exemple, et on prélève le sang dont on recueille le sérum. On s'assure que ce sérum ne contient pas d'anticorps anti-protéines humaines normales. En immunodiffusion le sérum obtenu donnera une ligne de précipitation avec un sérum d'origine humaine contenant de l'antigène NANBs.

L'Ac anti-NANBs peut être purifié et isolé, selon les méthodes connues de purification et d'isolement des gammaglobulines telles que les fractionnements à l'éthanol au sulfate d'ammonium ou au Rivanol.

Les anticorps NANBs contenus dans les gammaglobulines spécifiques préparées, et dont la présence peut être confirmée par les différents tests d'immuno-fluorescence, de CEP ou de DRI, sont capables d'agréger les particules virales NANB, y compris les virions complets associées à une activité ADN polymérase comme on peut le démontrer en faisant précipiter les virions complets marqués par des nucléotides radioactifs.

c) préparation du traceur.

A partir des anticorps anti-NANBs, ceux-ci sont couplés soit avec un traceur radioactif, par exemple à l'iode 125 pour obtenir un dosage radioimmunologique, soit avec un traceur enzymatique, par exemple avec la peroxydase pour obtenir un test immuno-enzymatique.

## Réalisation du test

Celui-ci est fait de manière analogue au test pour l'Ag NANBe.

II. Réalisation d'un test pour les Ac anti-NANBc.

L'Ag NANBc peut être purifié à partir du foie ou du sérum. L'Ag NANBc purifié va permettre de servir de base à la réalisation d'un test pour l'anti-NANBc par exemple par un DRI selon une méthode de compétition. Le test d'immunofluorescence indirecte en utilisant divers substrats provenant de divers malades et en testant des cas d'hépatite NANB de diverses origines a permis de montrer qu'il existe des variations d'Ag NANBc selon la souche du virus NANB en cause.

Deux principaux types d'Ag NANBc ont pu être à ce jour identifiés. Ils se comportent de manière analogue et les méthodes d'extraction sont identiques pour les 2 types principaux de virus NANB identifiés à ce jour, désignés par convention NANB1 et NANB2.

En immunofluorescence indirecte, utilisant comme substrat des coupes de foies infectés par un virus NANB (par exemple par le virus NANB1 ou NANB2), on détecte chez une population normale de donneurs de sang environ 10 % d'anticorps anti-NANBc1 et 5 % d'anticorps anti-NANBc2.

Avec la sensibilité du dosage radio-immunologique pour les anti-NANBc, on obtient un grand nombre de sujets sains chez qui on trouve des anticorps anti-NANBc1 ou 2 traduisant qu'ils ont été un moment donné de leur vie en contact avec ce virus et se sont immunisés sans faire de maladie.

Au total une minorité de tous ces donneurs de sang, soit 2 à 4 %, présente des anomalies détectables de la biologie hépatique sous forme de transaminases et/ou gamma GT au-dessus de 2 fois la valeur-seuil de la normale.

Les anticorps anti-NANBc apparaissent à la phase aiguë de la maladie dès le début de la montée des transaminases (en même temps qu'apparaît l'antigène NANBe) et ils persistent pendant toute la durée de la maladie et aussi pendant la convalescence et restent présents plusieurs années après la maladie. Il n'est donc pas douteux, vue la fréquence des expositions au virus NANB au cours de la vie, qu'une proportion relativement importante de la population ait des anticorps.

Le titre des anticorps est maximum à la phase aiguë de la maladie et si celle-ci guérit leur taux décroît.

9

Avec une méthode peu sensible telle que l'immunofluorescence indirecte, on garde une bonne valeur diagnostique, la présence des anticorps anti-NANBc traduisant une maladie en cours d'évolution ou étant en train de s'améliorer.

Grâce à l'immunofluorescence indirecte, on a retrouvé pour les virus NANB ce qui est connu pour les autres virus c'est-à-dire que la classe d'immunoglobulines qui supporte l'activité anticorps au début de la maladie est représentée par des immunoglobulines IgM, puis lorsque la maladie s'arrête et que le virus n'est plus produit, les anticorps IgM sont remplacés par des anticorps exclusivement IgG.

La mise en évidence d'anticorps anti-NANBc de classe IgM prend donc un intérêt diagnostique essentiel puisque ceux-ci vont traduire une infection en cours d'évolution. Si on se contente de rechercher les anti-NANBc IgM ceux-ci ne sont plus présents que chez à peu près 4 à 5 % des donneurs de sang. Au cours des hépatites aiguës, les anti-NANBc IgM ne persistent pas après la normalisation des transaminases dans la majorité des cas. Au cours des hépatites chroniques par contre, l'anti-NANBc IgM persiste. La valeur diagnostique est donc considérable.

On a également trouvé une relation quantitative entre la présence d'anti-NANBc à un titre élevé supérieur à 200 en DRI et une infection aiguë ou chronique toujours en évolution. En immunofluorescence indirecte, un titre supérieur ou égal au 40$^e$ traduit toujours que le virus se réplique et donc que le malade sera contagieux. En dosage radio-immunologique un titre supérieur au 500$^e$ traduit la réplication virale NANB actuelle ou persistante et s'accompagne toujours d'anti-NANBc IgM.

Pour atteindre le but recherché dans le diagnostic qui est de démontrer soit la présence du virus, c'est-à-dire son rôle dans une hépatite aiguë ou chronique et aussi d'autres syndromes comme nous l'avons vu, soit l'infectivité d'un sujet, en particulier celle d'un donneur de sang risquant de transmettre une hépatite NANB, on a deux possibilités : soit mettre en évidence des anti-NANBc IgM, soit titrer les anti-NANBc totaux détectés et admettre que tous les titres supérieurs au 200$^e$ et en tous cas à partir du 500$^e$ correspondent à une réplication du virus NANB.

L'objectif final étant d'avoir un test unique pour le repérage immédiat de tous les flacons de sang potentiellement infectieux, on peut réaliser un test où sur un support solide seront fixés d'une part l'Ag HBc permettant de détecter des anti-HBc et ainsi des sujets infectieux pour le virus HB et d'autre part des Ag NANBc 1 et 2 pour repérer les 2 principales classes de virus NANBc. La quantité de traceur anti-NANBc est calculée pour obtenir la sensibilité voulue correspondant au seuil recherché. On a ainsi un test polyvalent qui en 1$^{ère}$ intention repère tous les virus de la famille de l'hépatite responsables d'hépatites post transfusionnelles. Dans un 2$^e$ temps, on peut faire une analyse plus fine en séparant les cas anti-HBc-positifs qui sont dus au VHB des autres cas. Il ne faut pas non plus méconnaître l'existence d'infections doubles.

Pour la réalisation d'un test susceptible de repérer la classe IgM des anticorps anti-capsidiques du VHB et des virus NANB 1 et 2 on fixe sur le support solide, par exemple une bille de polystyrène des anticorps anti-IgM humaines d'origine animale (par exemple de mouton, lapin ou autres). Cette bille va donc représenter un piège pour toutes les IgM des sérums humains. Si on incube une telle bille avec un sérum, elle fixe les IgM circulants. Dans un 2$^e$ temps, on rajoute après lavage une quantité fixée d'antigène en l'occurrence soit de l'Ag NANBc1, NANBc2 ou HBc ou un de leurs mélanges le tout en dilution dans du sérum humain normal. Après incubation de 16 à 24 heures à la température ambiante on lave. S'il avait des IgM qui ont une activité anticorps contre l'Ag considéré, il y a eu attachement de l'Ag que l'on avait rajouté. Il reste alors à le révéler par addition d'un marqueur approprié, c'est-à-dire un anticorps anti-NANBc1, anti-NANBc2, ou anti-HBc, ou un mélange de ces anticorps, couplé à un traceur radioactif ou à un traceur enzymatique (par exemple le peroxydase, qui ensuite donne une réaction colorée lorsqu'elle est oxydée avec le periodate) et selon le cas on compte la radioactivité dans un compteur de radiation ou on apprécie la positivité par la réaction colorée à l'aide d'un photomètre qui la quantifie.

On peut également détecter des Ac anti-NANBc, avec un support sur lequel est fixé de l'Ag NANBc purifié, selon les méthodes (sandwich ou compétition) décrites ci-dessus.

Il est possible de réaliser des tests spécifiques pour chacun des Ag NANBc1 et NANBc2 mais il paraît plus logique d'envisager la réalisation d'un test unique pour les deux plus fréquentes infections à virus NANB. Pour cela on utilise un mélange des deux Ag (NANBc1 + NANBc2), fixé sur un support solide par mise en contact dans un tampon phosphate pendant une nuit à 4 °C. Après séchage et lavage, les sites éventuellement disponibles sur le support sont ensuite saturés avec une solution protéique telle que l'hémoglobuline ou l'albumine bovine. Le réactif constitué par le support ainsi enduit d'Ag va servir à la réalisation d'un test, par exemple selon la méthode de compétition. Il est incubé par exemple avec 200 µl de sérum à tester pendant 1 h à 37 °C. Après lavage on fait agir les Ac correspondants couplés au traceur, par exemple les Ac anti-NANBc radioactifs. La fixation maximum de ces Ac traduit la liberté des sites NANBc sur le support et donc que le sérum étudié ne contenait pas d'Ac anti-NANBc. Inversement, si on observe une réduction de plus de 50 % du nombre de coups/minute de radioactivité par rapport à un témoin, c'est que le sérum étudié contenait l'Ac anti-NANBc qui s'est fixé sur le support, muni d'antigène en rendant celui-ci inaccessible aux anticorps radioactifs ultérieurement mis à son contact.

III. Détection des infections hépatiques à virus NANB ou analogues à l'aide de l'Ag NANBe ou de l'Ac anti-NANBe.

L'intérêt d'un tel système provient du fait que l'Ag NANBe est l'Ag le plus profond des virus. C'est un Ag de groupe commun aux membres de la famille des virus d'hépatite qu'ils soient B ou NANBe. Une étude des hépatites post transfusionnelles, dans 6 centres différents : Lyon, Paris, Londres, Hannovre, Kiel, New York, a révélé que la fréquence de la détection de l'Ag NANBe au cours des hépatites post transfusionnelles variait de 60 à 100 % des cas avec une moyenne autour de 80 %. Compte tenu que la méthode utilisée était peu sensible (immunodiffusion) cela signifie que le système Ag/Ac NANBe utilisé est quasi constant dans les hépatites NANB post transfusionnelles. Ceci était d'autant plus surprenant que l'on pouvait s'attendre à des variations épidémiologiques dans des souches de virus NANB distinctes vu les régions très différentes. Il est en effet quasi démontré qu'il existe plusieurs types d'hépatites NANB.

Le fait que des virus de l'hépatite NANB distincts n'entraînent pas d'immunité croisée a conduit à penser que leurs antigènes d'enveloppe étaient différents mais pouvaient appartenir à la même famille de virus ayant en commun le même Ag NANBe. Ce phénomène a été démontré pour le virus NANB, le virus HB ainsi que pour un virus de l'hépatite d'une autre espèce animale, celui de la marmotte puisque pour les 3 virus on peut mettre en évidence Ag $HBe_3$ ou une réaction croisée avec $HBe_3$. Cette hypothèse de l'existence de plusieurs virus NANB possédant un même antigène de groupe NANBe a été confirmée lorsque des inoculums entraînant des hépatites NANB d'incubations très différentes et s'accompagnant de lésions ultrastructurales et de particules distinctes en microscopie électronique (souches H et F de SHIMIZU) ont été inoculées chez les chimpanzés (SHIMIZU, SCIENCE, 1980). L'étude des sérums des chimpanzés ainsi inoculés avec ces 2 souches de virus NANB distinctes a montré que l'Ag NANBe était détectable chez les chimpanzés infectés avec la souche F comme avec la souche H. La conclusion que l'Ag NANBe était un Ag de groupe de famille des virus de l'hépatite regroupant des virus de l'homme tel que le VHB, ou les virus NANB de souches différentes H et F et même le virus de la marmotte paraît donc bien établie.

Ceci confère donc un intérêt exceptionnel à cet Ag NANBe puisqu'il permet donc à priori de diagnostiquer tous les virus appartenant à ce même groupe, même des virus NANB ou autres Hépa DNA virus non encore découverts ou non encore apparus. L'intérêt de l'antigénicité croisée entre le virus HBe/3 du VHB et l'Ag NANBe du virus NANB n'est pas majeur pour le diagnostic des hépatites B puisqu'on dispose de très bons réactifs spécifiques pour la reconnaissance des hépatites à VHB. Par contre il n'en va pas de même dans le cas des hépatites NANB pour lesquelles aucun test diagnostique n'existait à ce jour.

Les difficultés de la sérologie NANB sont liées à l'existence fréquente au cours des hépatites NANB d'antigènes qui ne sont pas d'origine virale mais liés à l'hôte et qui sont présents à la phase aiguë de la maladie et induisent des anticorps. Ces Ag peuvent se rencontrer dans une pathologie et dans des conditions distinctes de l'hépatite NANB et sont à l'origine de nombreuses fausses réactions car ce sont des constituants normaux qui peuvent s'observer dans de très nombreuses circonstances cliniques avec atteintes du foie d'origines très diverses.

Ces phénomènes parasites expliquent qu'aucun test diagnostique reposant sur l'utilisation d'Ac naturels sélectionnés chez l'homme n'a pu constituer le point de départ d'un test satisfaisant jusqu'à ce jour. C'est donc une caractéristique essentielle de l'invention que de reposer sur l'utilisation d'Ag purifiés et d'Ac animaux spécifiques ou d'Ac humains monoclonaux ou polyclonaux de spécificité satisfaisante préparés par chromatographie d'affinité avec de l'Ag purifié.

Obtention des réactifs : Ag NANBe et Ac anti-NANBe nécessaires pour réaliser le test.

La réalisation d'un test spécifique pour l'Ag NANBe exige donc 1) la purification de l'Ag NANBe, 2) l'immunisation d'animaux afin d'obtenir des Ac spécifiques de titre élevé et d'affinité élevée, 3) sélection d'Ac monoclonaux ou polyclonaux monospécifiques. Grâce à ces anticorps il est possible de réaliser des tests de sensibilité variable croissante et spécifique tels que l'immunodiffusion, la contreélectrophorèse et surtout le dosage radio immunologique (DRI) et immunoenzymatique (ELISA).

Purification de l'Ag NANBe

Deux méthodes ont été mises au point pour la purification de l'Ag NANBe :

Première méthode

On prépare et purifie d'abord l'Ag NANBc à partir de foie infecté par un virus NANB. Sur l'Ag NANBc ainsi purifié on fait agir un agent détergent fort, c'est-à-dire un détergent ionique, en particulier anionique, tel que le SDS. On opère généralement en présence d'un réducteur tel que le dimercaptoethanol pour libérer l'Ag NANBe. Cet Ag est alors utilisé pour immuniser des animaux, par exemple des lapins pour obtenir des Ac anti-NANBe qui réagissent en immunofluorescence pour détecter l'Ag NANBe dans le noyau des hépatocytes infectés, ce qui confirme leur spécificité. Ces Ac spécifiques peuvent être utilisés pour détecter l'Ag NANBe dans le sérum par immunodiffusion et contreélectrophorèse. Cet AC purifié sert également de base à la réalisation du réactif selon l'invention permettant d'effectuer un test de DRI dit en sandwich ou bien un test immunoenzymatique qui sera détaillé plus loin.

Deuxième méthode

L'Ag NANBe sérique peut également être purifié par chromatographie d'absorption. On a découvert que l'Ag NANBe avait en particulier une affinité élective pour les gels d'héparine. Cela a servi de base à la mise au point d'une purification selon ce principe : dans un 1er temps, le sérum contenant l'Ag NANBe est appliqué sur une colonne de gel d'héparine où il se fixe et on constate que l'Ag NANBe est retenu sur la colonne ; dans un 2e temps on réalise l'élution et on recueille les fractions NANBe positives.

Immunisation des animaux (lapins, cobayes)

On opère par exemple par injections, faites en de multiples sites à chaque lapin de 300 μg de solution antigénique titrant 1/16 en CEP, après mélange avec de l'adjuvant complet de FREUND pour les 2e injections et pour les suivantes, à 1 mois d'intervalle, avec de l'adjuvant incomplet.

Obtention d'immuns sérums monospécifiques

Selon une première technique, on laisse incuber 2 h à 37 °C (ou 24 h à + 4 °C) les immuns sérums obtenus sur du sérum humain normal, polymérisé par traitement à la glutaraldéhyde et sur du broyat de foie humain normal également insolubilisé par la glutaraldéhyde. On répète l'opération jusqu'à disparition de toute réactivité contre des sérums ou extraits de foie normaux. Seule subsiste alors l'activité anti-NANBe.

Une autre voie d'obtention d'Ac monospécifique sera la préparation d'Ac monoclonaux selon la technique des hybridomes.

Une quatrième possibilité consiste à réaliser une absorption sélective des Ac anti-NANBe par chromatographie d'affinité sur un support revêtu d'Ag NANBe purifié, selon les méthodes connues.

Réalisation du test pour l'Ag et l'Ac NANBe

Ce système précipitant peut être détecté notamment pour :
— immunodiffusion (ID)
— contreélectrophorèse (CEP)
— dosage radio immunologique (DRI).

D'autres méthodes classiques de sérologie peuvent être employées grâce aux Ag purifiés et Ac monospécifiques qui ont été préparés, en particulier les tests d'hémagglutination, ou bien les dosages immunoenzymatiques.

Avec le réactif de l'invention, en fixant sur un support de l'Ag NANBe (ou de l'anticorps-anti-NANBe) il sera possible de détecter l'Ac anti-NANBe (ou de l'Ag NANBe) selon les méthodes de sandwich ou de compétition décrites ci-dessus.

IV. Cas particulier de la détection des donneurs de sang susceptibles de transmettre un virus de l'hépatite NANB, mais aussi tout autre virus apparenté Hepa-DNA virus.

A. Comme indiqué ci-dessus dans les généralités il s'agit d'un problème majeur par ses conséquences en raison :
1) de la fréquence du portage de ces virus par environ 3 à 10 % des donneurs de sang bénévoles, et une proportion allant jusqu'à 35 % pour les donneurs rémunérés et en pays d'endémie ;
2) de la gravité potentielle des hépatites NANB post transfusionnelles dont 50 % évoluent vers la chronicité avec finalement cirrhose du foie possible, et qui au total ont une morbidité et mortalité considérables.

B. Une des caractéristiques de l'invention est d'avoir précisé la possibilité de dépister les donneurs infectieux asymptomatiques par plusieurs tests sérologiques spécifiques permettant la détection de l'Ag NANBs, de l'Ag NANBe, et des Ac anti-NANBc/1 et 2, chacun des tests ayant son intérêt propre.

C. Une deuxième caractéristique de cette découverte est la caractérisation de la biologie et de la virologie des virus NANB, et la mise en évidence des différences très variables du comportement des marqueurs viraux NANB s, e et c, et une évolution très différente par rapport au virus HB.

a) L'Ag NANBs est beaucoup moins abondant que dans les infections à VHB par contre l'Ag NANBe lui est produit en quantités égales sinon plus importantes que dans l'hépatite B, et souvent supérieures à celles de l'Ag NANBs. Un test reposant sur l'Ag NANBe sera donc plus sensible dans certains cas.

b) Une autre caractéristique est le caractère fluctuant et recurrent mais hélas chronique des infections NANB avec épisodes de normalisation plus ou moins parfaite y compris des SGPT. On croit le sujet guéri puis il y a rechute. Les marqueurs NANBe et NANBs évoluent de façon semblable et disparaissent par épisodes du sérum :
α) en partie parce qu'il y a des cycles de synthèse avec des recrudescences et phases de quiescence,
β) en partie car les Ag sont masqués par les Ac anti-NANBe et anti-NANBs sous forme de complexes Ag/Ac, ceux-ci pouvant être infectieux. Dans le cas de complexes immuns masquant les Ag NANB/s et e, ou d'une brusque baisse de synthèse, les Ac anti-NANBc à titre élevé gardent toute leur valeur pour repérer un donneur dangereux. C'est même un de leurs intérêts essentiels.

γ) Il semble qu'inversement au virus HB il y ait aussi des cas avec de grandes quantités d'Ag NANBe, et NANBs accessoirement, où les anti-NANBc/1-2 sont à un titre très faible.

Au total les constatations conduisent à penser que le test le plus performant pour la détection du plus grand nombre de porteurs de virus serait un test qui permettrait à la fois la détection des différents marqueurs NANBs, NANBe et anti-NANBc. Un tel test est possible et nous allons en donner le principe.

Réalisation d'un test mixte ELISA et DRI en phase solide selon le principe du sandwich pour la détection simultanée des Ag NANB/s et e et du principe de la compétition pour les Ac anti-NANBc

On fixe d'abord les Ac anti-NANBe et anti-NANBs sur la bille qui piègera donc Ag NANBs et NANBe, on y rajoute en vue d'une phase ultérieure des Ac anti-NANBc/1-2, on veille à ce que les Ac anti-NANBe soient aussi fortement anti-NANBc et on peut même rajouter ceux-ci.

Le réactif ainsi réalisé va être mis à incuber avec le sérum à tester. S'il y a des Ag NANBs ou NANBe ceux-ci vont se fixer. On lave et on incube avec un mélange d'Ac anti-NANB/s + e, marqués à un traceur (enzymatique dans le cas d'un test ELISA).

On relève les cas positifs par photométrie.

Si le sérum est positif, il est infectieux et ne peut être transfusé. On pourra analyser ultérieurement quel est l'Ag en cause.

Si le sérum est négatif, ce n'est pas une preuve absolue qu'il n'est pas infectieux, simplement il est possible que les Ag NANBe et s ne soient pas détectables au seuil de sensibilité du test ELISA tel qu'il est construit.

Nous avons vu que ces Ag peuvent être moins abondants en phase de quiescence ou bien être masqués, mais le sang et le sérum restent infectieux.

On recherche alors les Ac anti-NANBc de la façon suivante. Au réactif qui porte les AC anti-NANBc/1 + 2, on rajoute une quantité déterminée d'Ag NANBc/1 + 2 dilué dans du sérum humain normal. On rajoute ensuite le sérum où l'on veut détecter les Ac anti-NANBc/1 + 2, et des Ac anti-NANBc/1 + 2 couplés à un traceur rédioactif, par exemple I 125. Après incubation, si le sérum à tester contient des Ac anti-NANBc/1 + 2, ceux-ci vont diminuer la fixation des Ac marqués du traceur sur le réactif auquel on a ajouté l'Ag NANBc/1 + 2.

Une réduction de plus de 50 % au nombre de coups comptés par spectromètre Gamma permet de conclure que le sérum testé est positif aux Ac anti-NANBc.

On va ainsi à nouveau retrouver un certain nombre de sérums infectieux marqués par la première phase du test, car le test sera calibré pour repérer les cas avec l'anti-NANBc/1 + 2 de titre supérieur à 1/500 signant l'existence d'un virus en réplication active.

Au total le test proposé est un test en deux temps ou un double test, l'un étant un test immunoenzymatique, l'autre un test radioimmunologique. Dans le cas particulier décrit ici,

la première phase est un test immunoenzymatique recherchant à la fois NANB/e et s selon la méthode du sandwich, et

la deuxième phase est un test radioimmunologique recherchant les anti-NANBc/1 + 2 selon un principe de compétition entre l'Ac à tester et l'Ac fixé au traceur.

D. Construction d'un réactif pour la détection du VHB et NANB.

Un autre aspect important de l'invention est donc la démonstration d'une réactivité croisée entre les Ag des virus B et NANB maximum pour HBe/3 et NANBe, intermédiaire HBc et NANBc/1 et 2, et très faible mais non nulle pour HBs et NANBs (en immunodiffusion avec Ac très purifiés).

Ces réactivités croisées ne sont pas suffisantes avec les réactifs commerciaux disponibles actuellement pour le VHB (sauf exception) pour détecter le virus NANB. En particulier en ce qui concerne la réactivité croisée maximum elle existe entre HBe/3 NANBe, mais pas avec HBe/1-2 qui représentent la base des tests DRI commercialisés jusqu'à ce jour.

Plusieurs tests pour le dépistage simultané des virus B et NANB peuvent être conçus.

1° Dépistage simultané des Ag HBs NANBs et NANBe (test sandwich)

a) On fixe sur le support solide les trois Ac anti-HBs, anti-NANBs, et anti-NANBe, en mélange.

On peut utiliser soit des Ac spécifiques d'animaux hyperimmunisés avec haute constante d'affinité, soit des Ac monoclonaux obtenus par la technique des hybridomes, soit des Ac humains polyclonaux rendus monospécifiques pour l'Ag voulu.

Si on a soin de sélectionner les Ac de classe IgM on peut augmenter la sensibilité du test.

b) L'incubation du sérum à tester se fait de façon standard.

c) Le traceur est également un mélange des 3 Ac couplés soit à $I_{125}$ en DRI soit à une enzyme (phosphatase alcaline ou peroxydase) en ELISA.

Là encore, l'utilisation d'IgM monoclonaux de haute avidité et de spécificité voulue peut augmenter les performances du test.

**0 074 986**

On effectue la révélation selon les techniques classiques.

2° Autre réactif pour la détection simultanée des Ag NANBs, HBs, NANBe et de l'Ac anti-NANBc

On fixe sur le support solide quatre Ac : anti-NANBs, anti-HBs, anti-NANBe et anti-NANBc/1 et 2. On met en contact avec une solution contenant de l'Ag NANBc dans du sérum humain normal puis avec le sérum à tester et on ajoute des Ac anti-NANBc/1-2 marqués par exemple à un traceur radioactif ($I_{125}$). Après lavage et révélation, si plus de 50 % de l'Ac anti-NANBc radio-actif est fixé au support c'est que le sérum était négatif pour les Ac anti-NANBc/1-2.

On met ensuite le réactif au contact d'une préparation d'anticorps anti-HBs, anti-NANBs et anti-NANBe couplés à un traceur différent de celui choisi pour la 1ère étape, donc ici enzymatique, peroxydase par exemple.

Après lavage et révélation, si les Ac marqués sont fixés sur le support c'est que le sérum à tester était positif pour l'un des Ag et donc infectieux.

Les exemples suivants illustrent l'invention sans toutefois la limiter.


Exemple 1


Réalisation d'un réactif avec l'Ac anti-NANBe.


On utilise des billes de polystyrène commercialisées par la Société PRECISION PLASTIC BALL (U.S.A.).

Les billes sont lavées dans l'éthanol pur avec agitation énergique pendant une nuit à température ambiante. Elles sont ensutie lavées à l'eau distillée et séchées en étuve à 37 °C.

Au départ d'un sérum reconnu positif à l'Ac anti-NANBe, on précipite les IgG par addition d'une solution de sulfate d'ammonium à 33 %. On purifie ensuite les gammaglobulines précipitées par chromatographie sur gel de DEAE Cellulose selon les méthodes connues de purification des gammaglobulines. Les fractions contenant les IgG sont diluées dans un tampon tris 0,01 M de pH 7,2 jusqu'à une concentration de 50 µg/ml. On laisse incuber les billes dans la solution diluée, en agitant doucement, pendant 18 heures à température ambiante.

On sépare les billes ainsi incubées puis on les fait incuber dans une solution d'albumine bovine à 5 % en tampon phosphate 0,02 M glycocolle 0,3 M de pH 7,5.

Après incubation pendant 4 heures à température ambiante, en agitant doucement de temps en temps, on sépare les billes, les lave à l'eau distillée et les sèche à 37 °C. On peut ensuite les conserver à température de 4 °C.

Le réactif constitué par les billes ainsi obtenues peut servir à la détection de l'antigène correspondant à l'anticorps fixé, selon une mthode dite « sandwich » qui est décrite ci-après.

Réalisation du test « sandwich » pour la détection d'un antigène.

Le principe du test est le suivant :

On incube 200 µl de sérum à tester avec une bille revêtue comme précédemment, pendant 18 heures à température ambiante.

Ensuite on lave la bille à l'eau distillée.

On incube alors la bille dans 200 µl d'un traceur radioactif pendant 4 heures à 37 °C. On lave la bille puis on effectue un comptage de radioactivité pendant une minute avec un spectromètre de radiations gamma type PACKARD par exemple. On procède par comparaison avec des sérums témoins reconnus négatifs à l'Ag NANBe.

Les sérums pour lesquels on observe un nombre de coups par minute deux fois supérieur au nombre de coups par minute observé pour les sérums témoins négatifs, sont considérés comme positifs.

Le tracteur radioactif est préparé de la façon suivante :

— des Ac anti-NANBe purifiés sont marqués avec l'iode radioactif par la chloramine T qu'on dilue ensuite de façon à obtenir entre $5.10^5$ et $10^6$ CMP/200 µl (soit la quantité que l'on applique sur une bille pendant la deuxième incubation).

Le diluant a la formule suivante :

— Tris HCl 0,01 M pH 7,5

— NaN$_3$ 0,1 %

— sérum humain normal (SHN) 10 %

— sérum de veau Fœtal (SVF) 50 %

Le marquage selon la méthode décrite par GREENWOOD est réalisé par exemple de la façon suivante :

On utilise 1,5 mCi d'iodure de sodium marqué à l'iode radioactif, que l'on ajoute à 50 µl d'IgG (anticorps anti-NANBe purifié) à 1 g/l en solution dans un tampon phosphate 0,05 M pH 7,5. On ajoute ensuite la chloramine T et le métabisulfite selon la méthode de GREENWOOD.

On passe ensuite la solution obtenue sur une colonne DOWEX 2X 8 000 (marque commerciale) de

14

diamètre de gel 100-200 mesh. On utilise 3 ml de gel. On recueille 3 ml d'éluat que l'on passe sur une colonne de SEPHADEX G 25 % (marque commerciale) (10 ml de gel). On récupère les cinq premiers millilitres élués après le volume mort de la colonne. Ils contiennent l'anticorps marqué à l'iode radioactif.

Exemple 2

Réalisation d'un réactif pour les Ac anti-NANBc

Purification des Ag NANBcl et NANBc2. (et HBc)

a) purification à partir du foie

Un homogénat de foie est préparé par broyage à partir d'un foie d'autopsie d'un malade décédé d'hépatite NANB et dont le foie a été trouvé positif en immunofluorescence directe avec un anti-NANBc conjugué pour l'Ag correspondant NANBc1 ou 2.

Le broyage est effectué en présente d'un tampon Tris HCl 0,05 M, pH = 7,6 + 0,25 M Sucrose + 0,006 M Mg Cl$_2$, +0,025 M KCl. Le broyat brut est filtré. L'homogénat est centrifugé 20 minutes à 4 000 g (+4 °C). Le culot est resuspendu dans le même tampon, homogénéisé et centrifugé 20 minutes à 4 000 g. Cette opération est recommandée 2 fois. Le culot final est resuspendu dans un tampon Tris -HCl pH 7, 6, 0,05 M + NaCl 0,15 M + détergent non ionique NP 40 0,1 % + β mercaptoéthanol 0,02 % + pronase E (70 000 PUK)g MERCK) à concentration finale de 0,1 %.

Après 1 heure à 37 °C et 1 nuit à +4 °C, on soumet à une centrifugation pendant 20 minutes à 10 000 RPM. On élimine le culot. Le surnageant est centrifugé 16 heures à 27 000 RPM sur 10 ml d'un gradient de sucrose 10-20 % en tampon Tris-EDTA 1 mM + NaCl 1M. Les culots sont repris dans CsCl (d = 1,22 g/cm³) en tampon Tris EDTA 10 mM + BSA 0,1 % (volume final : 5 ml) et on dépose sur 28 ml d'un gradient de Cs Cl 1,28-1,43 g/cm³, le tube est complété avec CsCl (1,18 g/cm³) et centrifugé 40 heures à +4 °C et 25 000 RPM.

Le gradient est récolté par le fond du tube en fractions de 1,2 ml qui sont dialysées après lecture de l'index sur réfractomètre. Les fractions positives sont rassemblées et centrifugées sur coussin de sucrose 10-20 %.

On peut procéder ensuite à une nouvelle ultracentrifugation en gradient de sucrose. Les fractions concentrées sont mises sur un gradient continu de 15 à 55 % de sucrose préparé en tampon TSA (tampon Tris 0,05 Hcl pH 7, 6, 0,15 M NaCl et 0,02 % Na3) et ultracentrifugées pendant 18 heures à 24 000 t/mn à 4° dans le rotor SW27.

Des fractions d'1,5 ml sont ensuite collectées à partir du bas du tube puis testées pour la DNA polymérase et pour les Ag NANBc. La densité de chaque fraction est déterminée par l'index de réfraction dans un réfractomètre ABBE. Toutes les fractions sont conservées à 4°.

C'est ce corps purifié et/ou plutôt le mélange des fractions ainsi obtenues pour NANBc1 et NANBc2 (ainsi que pour l'Ag HBc éventuellement) qui pourra être utilisé soit pour être fixé directement sur une bille pour détecter les anticorps anti-NANBc, soit pour être rajouté dans un 2ème temps si on réalise le test IgM.

Purification des antigènes capsidiques NANBc1 et 2 (ou HBc) à partri du sérum.

Pour cela, on choisit un sérum NANBe très positif provenant de donneurs de sang ou de malades et si possible de sujets immunodéprimés tels que les hémodialysés et des leucémiques qui sont réputés pour avoir de grandes quantités de virus circulant, ayant un titre très élevé d'Ag NANBe par exemple 1/8 en CEP ainsi que l'Ag NANBs, et surtout pour lesquels en faisant la réaction d'ADN polymérase sur un culot d'ultracentrifugation du sérum ainsi repéré, on trouve une activité ADN polymérase. Après avoir ainsi identifié les sujets qui peuvent représenter des sources intéressantes de virus complet, on obtient chez eux une quantité importante de plasma ou sérum prélevé soit lors d'un don de sang, soit lors d'une plasmaphérèse suivie de recalcification du plasma. On procède ensuite à une ultracentrifugation.

On commence en partant de 18,5 ml de sérum qui sont clarifiés puis qui sont centrifugés à travers 20 ml d'un gradient continu de sucrose 10-20 % sur rotor SW27, 20 heures à 25 000 RMP puis le culot obtenu est repris dans du chlorure de césium.

Les fractions sont récupérées à partir du bas du tube qui est perforé. On recueille des fractions de 0,5 ml sur lesquelles on va mesurer l'index de réfraction dans le réfractomètre ABBE l'activité ADN polymérase et l'Ag NANBs. On va sélectionner les fractions positives pour l'activité ADN polymérase si on s'assure en microscopie électronique qu'elles contiennent bien la particule virale complète à double membrane correspondant au virion complet.

Pour obtenir l'Ag NANBc qui est représenté par le centre de ces virus complets on reprend cette fraction et la recentrifuge, après l'avoir mélangée à 2 volumes de solution riche en particules virales complètes avec 1 volume de détergent vendu sous la marque commerciale Nonitet P40 10 % (Shell Company), sur un gradient 15-55 % de sucrose en tampon TSA comme décrit plus haut pour la purification à partir du foie. Les fractions sont testées pour l'activité ADN polymérase et les Ag capsidiques NANBc par CEP. Les fractions positives en Ag sont récupérées et traitées comme décrit ci-dessus. Elles serviront à la préparation du réactif.

## Réalisation du réactif

a) test anti-NANBc simple
Lavage des billes de polystyrène vierges (cf test HBC ci-dessus).

b) couplage par adsorption de l'Ag NANBc sur les billes. Une solution en proportions convenables soit d'Ag NANBc1 ou 2 soit mélangée à de l'Ag HBC est fixée sur les billes par incubation dans l'Ag par agitation 12 à 18 heures à température ambiante.

c) saturation des billes, l'excès d'Ag après couplage est éliminé et sans lavage préalable on ajoute aux billes une solution tampon pH 7.5 phosphate 0,02 M glycocolle 0,3 M albumine bovine 5 %. On incube 4 heures à température ambiante, on agite doucement à intervalles réguliers.

d) les billes sont lavées à l'eau distillée, elles sont séchées à 37° et conservées à 4°. Ces billes couplées à l'Ag sont prêtes à être utilisées pour la détection de l'anti-NANBc correspondant ou bien des différents anticorps anti-capsides.

e) préparation du traceur : il s'agit d'anticorps anti-NANBc1, 2 et/ou anti-HBc. On prend 50 µl d'IgG avec l'activité anticorps choisie à 1 µg/µl dans du tampon phosphate à 0,05 M pH 7.5, soit 50 µg de protéines. On dispose de 1,5 mCi de iodure de sodium contenant I 125.

50 l de phosphate 0,5 M pH 7.5 sont mélangés avec la chloramine T et du métabisulfite selon la technique de Greenwood.

A la fin du marquage, passage immédiat sur colonne Dowex (marque commerciale 2 × 8 cm ⌀ 100 à 200 mesh (3 ml de gel). On récupère 3 ml puis on passe sur colonne Sephadex G 25 (marque commerciale) soit 10 ml de gel. Les 5 premiers ml après le volume mort de la colonne sont récupérés et contiennent l'anticorps marqué à l'iode radioactif séparé de l'iode libre. Le traceur est dilué dans un diluant traceur (cf test HBe ci-dessus) pour l'analyse d'une dilution dans ce milieu de façon à obtenir entre 500 000 et 1 000 000 de coups par minute pour 200 µl.

## Réalisation du test proprement dit

La bille recouverte d'Ag NANBc est incubée avec 200 µl de sérum à tester pendant 18 heures à température ambiante.

Ensuite on lave à l'eau distillée abondamment.

On incube avec 200 µl de traceur radioactif contenant donc 500 000 à 1 000 000 de CPM, l'incubation a lieu pendant 4 heures à 37° au bain-marie.

On lave à nouveau comme précédemment décrit et on compte 1 minute dans un spectromètre de radiation gamma Packard.

Calcul des positifs ; il se fait par comparaison avec les résultats obtenus dans des sérums négatifs. Un sérum est positif si le nombre de coups/minute est inférieur à 50 % de celui obtenu pour la moyenne des sérums témoins négatifs soit une inhibition de plus de 50 % de la radioactivité. Au lieu d'utiliser un traceur radioactif, on peut utiliser un traceur enzymatique représenté par un marquage des anticorps à la peroxydase (HRP), RZ 30 Boehringer Manrheim bH West Germany. Ce couplage est fait selon la méthode de Nakane, Journal Histochemistry Cytochemistry, 22 : 1 084-1 091 (1974).

## Test pour la détermination des anti NANBc IgM

Une bille de polystyrène est couplée initialement avec l'anticorps anti-IgM humaines, de mouton ou de lapin, ce sont les mêmes conditions de couplage de la bille que pour les anticorps anti-NANBe (voir exemple 1).

Ensuite avec 200 µl de sérum dilués au 500ème dans un tampon 0,01 PBS pH 7.2 0,05 Tween-20 (PBS TW) on lave la bille. (Tween est une marque commerciale).

On rajoute 200 µl d'une solution de sérum humain normal contenant des antigènes NANBc1, 2 et HBc séparément ou ensemble. On incube à nouveau 16 à 24 heures à la température ambiante.

Nouveau lavage, nouvelle incubation avec le traceur dilué convenablement, incubation 4 heures à 37°. Lavage.

Dans ce cas le traceur n'est pas simplement réalisé par le marquage des anticorps complets à l'iode radioactif, il s'agit d'anticorps partiellement coupés que l'on appelle fragments F (ab') 2 ceci afin d'éviter une réaction non spécifique avec le facteur rhumatoïde qui est une anti-IgM non spécifique de l'hépatite. Donc les anticorps anti-NANBc de classe IgG sont transformés en fragments F (ab') 2 par la méthode de Rossi (Nature 223 : 837, 1969).

Pour cela simplement les IgG sont digérés avec la pepsine (2 700 ug/mg à pH 4.3 pendant 20 h) l'action est stoppée en ajoutant du Tris hydroxyméthyl aminoéthane à pH8.

Les petits peptides et les fragments F (c') sont enlevés par filtration sur gel sur Sephadex G50. Les IgG non digérés par l'action de la pepsine sont éliminés par absorption sur une colonne immunoabsorbante couplée à un anticorps anti-Gamma F (c) humain couplé à du Sepharose 4B par du bromure de cyanogène.

Préparation des anticorps anti-NANBc

Les anticorps anti-NANBc 1 et 2 de titre très élevé peuvent être soit obtenus chez des malades dont le sérum apparaît en immunofluorescence avoir des titres supérieurs au 1/100ème en IF indirecte, soit chez des animaux immunisés avec les Ag NANBc 1 et 2. Ces anticorps peuvent soit être directement conjugués au traceur radioactif ou enzymatique, soit être utilisés pour la préparation des fragments F (ab') 2. Inversement, on peut utiliser les Ag NANBc1 NANBc2 purifiés et immuniser des lapins ou cobayes avec ces antigènes comme décrit pour la préparation des anti-NANBe à partir des Ag NANBe purifiés.

Exemple 3

Purification de l'Ag NANBs

L'Ag NANBs peut être purifié par exemple de la façon suivante.

a) Choix des plasmas positifs pour l'antigène NANBs

Les sujets ayant été trouvés porteurs d'antigène NANBe au cours de programmes de détection systématique de cet antigène et de transaminases chez des donneurs de sang, ou bien à l'occasion de perturbations clinique et/ou biologiques, hépatiques, en particulier, des transaminases (notamment TGP) ou des gammas glutamyl transpeptidases chez des consultants plus ou moins symptomatiques, sont également testés pour l'antigène NANBs en immuno-diffusion et contre-électrophorèse.

Nous avons observé que 5 % des donneurs de sang environ ont des TGP 2 fois supérieure à la normale, 80 % de ces mêmes individus sont positifs pour l'Ag NANBe et plus de 15 % pour l'Ag NANBs. Les sujets trouvés porteurs d'un titre suffisant d'antigène NANBs 1/4 ou 1/8 par contre-électrophorèse si possible, font l'objet d'un prélèvement de sang ou de plasma. Pour la préparation du vaccin on s'adressera de préférence à des sujets apparemment sains, les perturbations hépatiques modérées signalées ci-dessus n'étant pas rédhibitoires en elles-mêmes.

b) Purification de l'antigène NANBs par chromatographie sur gel de sépharose CL 4-B (Pharmacia, UPSALA, Suède)

Une colonne de chromatographie est équilibrée avec du tampon tris 0,05 M HCl 0,15 M, pH 7,6. On applique sur la colonne une fraction sérique enrichie d'antigène NANBs (par une précipitation préalable au sulfate d'ammonium à 60 % suivie de dialyse contre le même tampon), une telle fraction se 5 ml réagissant fortement pour l'antigène NANBs est posée sur la colonne. L'élution se fait avec le même tampon et le débit est réglé à 25 ml par heure. Des fractions de 5 ml sont collectées et testées pour l'antigène NANBs et pour l'antigène NANBe en immunodiffusion et C.E.P. (contre-électrophorèse).

L'analyse des fractions recueillies fait apparaître 5 pics de protéines par mesure de la densité optique à 280 nm. L'antigène NANBs est détecté dans les premières fractions, immédiatement après le volume d'exclusion de la colonne. C'est dans ces premières fractions que l'on observe le tire le plus élevé d'antigènes NANBs, atteignant 1/32 en C.E.P. Les fractions ultérieures, ont un titre plus bas. Et elles sont également positives pour l'antigène NANBe. Les fractions NANBs très positives sont réunies et repassées une nouvelle fois sur la colonne pour augmenter la qualité de la séparation.

On réunit à nouveau les fractions NANBs et on les concentre pour les soumettre éventuellement à des étapes de purification ultérieures. On peut également faire suivre plusieurs séries de colonnes différentes, en particulier avec le Sépharose® 6 200 SUPERFINE (Pharmacia) ou l'Ag NANBs sort essentiellement dans les 2ème et 3ème pics de protéines des fractions éluées.

L'antigène NANBs peut également être isolé par ultracentrifugation en gradient de densité de sucrose ou de chlorure de césium.

Il peut également être purifié par chromatographie d'affinité.Pour cela on peut utiliser divers supports tels que le Sépharose, ou un support permettant une utilisation en « batch » au lieu de colonne comme la MAGNOGEL (marque commerciale) de l'Industrie Biologique Française ; dans ce cas on incube les Ag anti-NANBs après activation du gel avec la glutaraldéhyde pour réaliser l'immunoabsorbant.

a) Préparation d'un immuno absorbant Sépharose®, anti-NANBs :
La fraction gammaglobulinique d'un sérum trouvé fortement positif en anticorps anti-NANBs est préparé par précipitation avec du sultate d'ammonium pH = 7 à 33 % de concentration finale. Le précipité est redissout en tampon 0,1 M NaHCO$_3$ et dialysé vis-à-vis d'une solution 0,5 M NaCl-0,1 M, NaHCO$_3$. La concentration par rapport au sérum de départ est de 3 fois. Les gammaglobulines anti-NANBs sont couplées à du Sépharose 4B (marque commerciale) activé au bromure de cyanogène (4B CNBR-Pharmacia — UPSALA — Suède), selon le procédé décrit par CUATRECASAS et AFINSEN dans ANN. Rev. Biochem. 40 : 259, 1971. 7 g de Sépharose 4B (marque commercial) activé au CNBr sont mis à gonfler et sont lavés sur un filtre en verre avec une solution 0,001 M HCl pendant 30 minutes. Immédiatement après lavage, le gel est mélangé avec 200 mg de gammaglobulines anti-NANBs en solution bicarbonatée et on agite pendant 2 heures à température ambiante.

Le gel est ensuite lavé avec 600 ml de la solution 0,1 M NaHCO₃ contenant 0,5 M NaCl, et traité avec 50 ml d'une solution d'éthanolamine 1 M pH = 8 pendant 2 heures à 25 °C.

Le Sépharose ainsi couplé est ultérieurement lavé alternativement avec un tampon IM NaCl, 0,1 M acétate, pH = 4 et un tampon 1M NaCl, 0,1 M borate, pH = 8,4. Le dernier lavage est fait en tampon 0,1 M borate, pH = 8,4 contenant 0,5 M de Nacl et 0,005 M d'EDTA.

b) Dans un autre mode d'exécution du procédé on a utilisé un immuno absorbant MAGNOGEL (marque commerciale), active à la glutaraldéhyde et couplé avec les immunoglobulines anti-NANBs.

c) Isolement de l'antigène NANBs :

Une colonne de 2 × 11 cm de Sépharose® couplée à l'anti-NANBs est utilisée. La colonne est équilibrée avec le tampon à température ambiante. On ajoute 25 ml de la solution concentrée contenant de l'antigène NANBs de titre élevé. Cette solution est diluée au demi avec le tampon et on laisse 1 heure à 37 °C pour favoriser l'adsorption. La colonne est ensuite placée à + 4 °C et lavée avec le tampon borate jusqu'à ce que la densité optique des fractions devienne nulle. L'antigène NANBs est élué de la colonne par un tampon phosphate 0,1 M, pH 10,8. Les fractions de 5 ml sont recueillies et la densité optique à 280 nm mesurée. On ramène immédiatement à un pH physiologique par addition NANBs et toutes les fractions positives sont réunies et concentrées par ultrafiltration sur filtre AMICON (marque commerciale) retenant toutes les protéines ayant un poids moléculaire supérieure à 30 000.

Toutes autres conditions de couplage et d'élution en particulier celles préconisées par le fabricant du Sépharose 4B CNBR peuvent être utilisées pour cette méthode de purification. (Sépharose est une marque commerciale)

## Exemple 4

### Purification de l'Ag NANBe par la méthode au gel d'héparine

On sélectionne des sérums positifs en Ag NANBe en ID et en CEP. Après filtration sur Millipore® (diamètre des pores 0,45 micromètres) pour clarification, on soumet à une ultracentrifugation pendant 2 heures à 300 000 g. On recueille les surnageants (les culots peuvent être utilisés pour isoler les virus NANB recherchés par l'activité de DNA polymérase).

On ajoute au surnageant une solution à 33 % de sulfate d'ammonium (SA) à raison de 0,5 vol. de solution de sulfate d'ammonium pour 1 volume de surnageant pour une concentration en S.A. de 33 % afin de précipiter notamment les gammaglobulines. On élimine le précipité par centrifugation (on peut rechercher éventuellement dans ce précipité la présence d'anticorps anti-NANB). Au surnageant on ajoute du S.A. jusqu'à concentration de 70 % (en ajoutant du S.A. cristallisé à la solution à 33 %). On centrifuge, élimine le surnageant, et reprend le culot par le TSA. On élimine le sulfate d'ammonium par dialyse contre le TSA. On vérifie par CEP et ID que le produit obtenu a un titre satisfaisant en Ag NANBe.

On chromatographie la fraction ainsi obtenue sur une colonne remplie par 200 ml d'héparine Ultragel IBF (marque commerciale pour une héparine fixée sur gel d'agarose) équilibrée en tampon T1 (Tris 0,05 M, NaCl 0,15 M, NaN₃ 0,02 %, CaCl₂ 0,025 M, pH 7,6), en déposant 5 ml de ladite fraction. On élue par le tampon T1 (100 ml environ) à faible débit (0,2 ml/minute) puis on lave (débit 1 ml/minute) avec 200 ml de TI puis 100 ml de tampon T2 (Tris 0,05 M, NaCl 0,15 M, NaN₃ 0,02 %, EDTA 0,01 M, pH 7.6), et élimine l'éluat. On élue ensuite l'Ag NANBe par un gradient de solution aqueuse de chlorure de sodium dans le tampon T2 obtenu en ajoutant progressivement (0,5 ml/minute) une solution Tris 0,05 M EDTA 0,01 M NaCl 2,5 M dans un flacon, muni d'un agitateur, contenant 300 ml de tampon T2. On collecte des fractions de 10 ml (50 fractions environ), en évaluant la concentration en NaCl au réfractomètre d'ABBE. Les gradients allaient de 0,15 M à 1,2 M NaCl. Les fractions sont dialysées contre du tampon TSA, puis reconcentrées par ultrafiltration (limite 10 000 daltons) à environ 1 ml. On recherche la présence d'Ag NANBe par ID et CEP, qui est observée principalement dans les fractions correspondant à 0,3-0,7 M NaCl. On réunit ces fractions et obtient ainsi une fraction purifiée d'Ag NANBe pouvant être utilisée par exemple pour réaliser un réactif selon l'invention destiné à détecter la présence d'Ac-NANBe.

## Exemple 5

### Obtention d'Ag NANBe au départ de l'Ag NANBc

On utilise comme produit de départ la fraction positive en Ag NANBc obtenue à l'exemple 2 a) aprés ultracentrifugation en gradient de sucrose à 4 ml de cette fraction on ajoute 1,33 ml de β-mercaptoéthanol à 0,5 % et 1,33 ml de dodécyl sulfate de sodium (SDS) à 0,5 %, soit une concentration finale de 0,1 % en β-mercaptoéthanol et 0,1 % en SDS, on laisse incuber pendant 2 h 1/2 à 37 °C, puis on dialyse pendant 20 heures à + 4 °C contre du tampon PBS.

On obtient une fraction riche en Ag NANBe qui peut être utilisée par exemple pour immuniser des lapins. Pour cela on le filtre sur membrane millipore stérile 0,22 micron préalablement saturée par le sérum de lapin normal.

**0 074 986**

Exemple 6

Procédé de purification de l'antigène NANBs en partant de la propriété antigénique croisée faible avec l'Ag HBs.

Comme indiqué ci-dessus, il existe une antigénicité croisée notable entre les virus NANB et HB en ce qui concerne les Ag profonds : e et c. Il existe également une faible antigénicité croisée non détectable par les réactifs commerciaux y compris les DRI au niveau des Ag d'enveloppe des virus. Cette règle qui s'observe pour les virus de l'hépatite de la marmotte et le VHB est également vraie pour les virus NANB. Bien que faible, la réactivité croisée peut servir de préparation de l'Ag NANBs.

On part de plasmas d'Ag HBs de titre très élevé supérieur ou égal à $10^7$ en DRI de sous-types ay et ad et positifs à l'Ag et Hbe. Ensuite, on purifie ces 2 types d'Ag HBs par les méthodes classiques telles que décrites en particulier dans Am. J. Dis. Children, 1972, p. 304. L'Ag HBs purifié de sous-type ay est utilisé pour immuniser des lapins selon le schéma d'immunisation classique pour immuniser des lapins selon le schéma d'immunisation classique déjà précité. Dans un 2ème temps, on va stimuler les lapins qui auront développé des Ac anti-HBs (anti-ay) avec de l'HBs purifié ad. Le résultat est l'obtention d'Ac anti-a de titre élevé. On s'est aperçu qu'un certain nombre de ces Ac anti HBs anti-a de très haut titre étaient capables de réagir, vis-à-vis des Ag NANBs en identité partielle avec des Ac anti-NANBs cf schéma. Ceci montre qu'il y a une parenté entre la spécificité a de l'Ag HBs et l'Ag NANBs. Les anticorps anti-a, ainsi sélectionnés, vont être utilisés pour réaliser un grand nombre d'arcs de précipitation en contre-électrophorèse classique sur plaque avec des Ag NANBs sélectionnés comme précédemment indiqué, et bien sûr négatifs pour l'Ag HBs. Les plaques de gélose de la contre-électrophorèse sont lavées en tampon PBS puis dans de l'eau distillée en finale. Les lignes de précipitation sont découpées au scapel et un grand nombre sont réunies, homogénéisées et mélangées à de l'adjuvant de FREUND. Ce mélange Ag NANBs, Ac de lapin anti-HBs/anti-a va être utilisé pour immuniser les lapins qui réagiront contre le seul Ag NANBs car les lapins ne réagissent pas contre leurs propres gammaglobulines. Après simulation adéquate des lapins, on réussit à obtenir chez certains d'entre eux une réponse anti-NANBs qui n'est plus anti-HBs. Ces Ac sont repris après purification sur DEAE cellulose des IgG pour réaliser une chromatographie d'affinité et celle-ci permet à partir de fractions Ag NANBs de purifier cet Ag selon les procédés déjà décrits. Le nouvel Ag NANBs de pureté accrue ainsi préparé est à nouveau utilisé pour immuniser les lapins qui serviront de source d'Ac anti-NANBs de plus grande pureté et de haute affinité y compris grâce à la sélection de fractions Ac de classe IgM qui sont particulièrement utiles dans la réalisation de tests sensibles visant à détecter l'Ag NANBs en DRI.

L'Ag NANBs le plus purifié peut également être utilisé pour préparer des Ac monoclonaux anti-NANBs par immunisation de souris puis fusion des lymphocytes spléniques avec des lymphocytes myélomateux selon la technique des hybridomes décrite par MILSTEIN et ses collaborateurs. La faculté de produire les Ac anti-NANBs par les clones cellulaires qui seront sélectionnés est faite par les tests d'immuno-fluorescence indirecte sur des substrats de foie Ag NANBs ou bien par contre-électrophorese ou précipitation DRI (avec des Ag NANBs et billes à l'Ag anti-NANBs).

La méthode décrite ici pour l'Ag NANBs peut être également utilisée pour la purification des Ag NANBe en partant de la réactivité croisée avec l'Ag HBe/3 et aussi pour l'Ag NANBc en partant de la réaction croisée avec HBC. Dans ces 2 cas on peut déjà directement partir d'Ac-anti HBe/3 par exemple pour faire une première chromatographie d'affinité, sur un support couplé aux anti HBe/3, permettant d'obtenir des fractions concentrées d'Ag NANBe.

En immunisant des lapins avec de l'Ag HBe/3 les AC de lapins anti HBe/3 servent à préparer des lignes de précipitation avec Ag/NANBe, avec lesquelles on immunisera des lapins neufs et restimulera des lapins anti-HBe/3.

On peut aussi utiliser ces modèles naturels d'hépatite virale tels que ceux du chimpanzé et de la marmotte en simplifiant les purifications car on travaille en système homologué.

1) On part de sérum Ag HBs de chimpanzé ay et ad. Le sérum non purifié sert à hyperimmuniser de l'adjuvant l'animal qui fera des Ac anti HBs/anti-a.

2) Les lignes de précipitation anti-a/Ag NANBs (ou toute autre préparation d'Ag NANBs purifié) servent à stimuler l'animal. On obtient ainsi des Ac anti-NANBs. Pour le chimpanzé on peut restimuler avec de l'Ag NANBs de chimpanzé obtenu en transmettant du virus NANB à un animal ou en stimulant tout plasma de chimpanzé Ag NANBs détecté chez des animaux naturellement ou expérimentalement infectés par le virus NANB.

On peut faire la même opération avec l'Ag HBE/3 pour aboutir à des Ac anti-NANBe.

Enfin au lieu d'utiliser le chimpanzé on peut utiliser la marmotte, également naturellement infectée avec un virus WHV apparenté aux virus B et NANB et ayant aussi des Ag, e, c, s qui croisent dans les mêmes proportions.

Des marmottes sont hyperimmunisées en Ac anti-WHs ou Ac anti-WHe avec des Ag WHs et WHe purifiés à partir de sérum de marmottes. On stimule ensuite la formation d'Ac contre le déterminant commun entre le virus WHV et NANB par les artifices décrits ci-dessus pour les Ag e, s ou c.

# 0 074 986

## Exemple 7

L'invention a également pour objet la préparation d'Ac monoclonaux contre les déterminants communs aux Ag de surface des virus NANB et HB, en utilisant la propriété d'antigénicité croisée avec les Ag correspondants du VHB.

Le protocole général suivi peut être celui de la préparation de tous les Ac monoclonaux ; voir par exemple « Monoclonal antibodies, Hybridomas : New dimension of biological analysis », Kenneth Robert, MAC-KEARN Thomas, BOCH Tol. K., Plenum Press, New York, London, 1980, p. 3 à 30.

On stimule un animal successivement avec les Ags des virus de l'hépatite B et NANB, on vérifie que l'animal a bien développé des anticorps contre VHB et VHNANB, on stimule à nouveau ledit animal pour injection d'un desdits Ags (par exemple Ag NANBs), puis, après le temps nécessaire pour l'élaboration de la réponse anticorps, on recueille des lymphocytes dudit animal, les fusionne avec une lignée lymphocytaire myélomateuse, réalise des cultures des hybrides obtenus, et sélectionne par clonage les hybrides produisant des anticorps à la fois anti-HBs et anti-NANBs.

On immunise une souris avec par exemple 5-15 microgrammes d'Ag NANBs partiellement purifié. 10 à 20 jours plus tard on stimule avec 5-15 microgrammes d'Ag HBs purifié.

On s'assure 7-15 jours plus tard que la souris a bien développé desAc anti-NANBs (vérification faite par immunofluorescence sur une biopsie de foie avec Ag NANBs cytoplasmique ou membranaire ou bien par DRI comme décrit) mais aussi qu'elle a fait des Ac anti-HBs (vérification faite en utilisant les réactifs commerciaux).

Une formation d'anti-HBs accélérée confirme bien qu'il y a eu un effet de stimulation anamnestique vis-à-vis de déterminants Ags communs aux Ag de surface des virus HB et NANB.

On fait alors une troisième injection d'Ag NANBs. 48 heures plus tard on prélève la rate des souris et recueille les lymphocytes. Ceux-ci sont fusionnés avec une lignée lymphocytaire myélomateuse des souris, et des hybrides sont obtenus, de façon connue. On réalise des cultures dans des microplaques de façon connue, de ces hybrides. Quand les cultures se sont bien établies, on teste les surnageants de chaque puits de microplaque pour la présence d'Ac anti-HBs (par réactif commercial) et d'Ac anti-NANBs par immunofluorescence et/ou DRI. Les lymphocytes des puits produisant des Ac à la fois anti-HBs et anti-NANBs sont alors clonés de façon connue.

Les cultures sont à nouveau propagées.

On s'assure, après plusieurs repiquages si nécessaire, que l'on a bien obtenu :

a) des Ac monoclonaux provenant d'un seul clone de cellule par exemple exclusivement de la même classe d'immunoglobuline IgG ou IgM,

b) que ce clone produit bien des Ac à la fois anti-NANBs et anti-HBs.

Les Ac sont dirigés contre les déterminants communs au VHB et au virus NANB.

Les anticorps ainsi obtenus sont alors fixés par couplage sur des billes de polystyrène, comme décrit ci-dessus.

Le réactif unique ainsi obtenu permet de détecter aussi bien les Ags de virus HB que les Ags de virus NANB, de même que les anticorps correspondants.

## Exemple 8

Purification de l'Ag NANBs, par chromatographie d'absorption sur héparinogel.

On sélectionne des sérums positifs en Ag NANBs que l'on concentre par précipitation au sulfate d'ammonium à 60 %. Après dialyse, la préparation concentrée est chromatographiée sur une colonne contenant 200 ml d'héparine Ultragel (marque commerciale) IBF équilibre en tampon $T_1$ (voir exemple 4), avec un écoulement lent de 0,2 ml/minute. La colonne est alors lavée avec 200 ml de $T_1$ puis par 100 ml de $T_2$ (conforme exemple 4). On élue ensuite l'Ag NANBs avec un gradient de NaCl dans le tampon $T_2$ obtenu en ajoutant progressivement (0,5 ml/minute) une solution Tris 0,05 M EDTA 0,01 M NaCl 2,5 M dans un flacon de 300 ml de $T_2$.

On recueille des fractions de 10 ml, les gradients en NaCl, allant de 0,15 M à 1,2 M étant mesurés au réfractomètre d'ABBE. Les fractions sont dialysées contre du tampon TSA puis concentrées à 1 ml par ultrafiltration (limite 10 000 daltons). On recueille les fractions positives en Ag NANBs, correspondant aux fractions 0,25-0,40 M et 0,60-0,80 M. Ces fractions à haute teneur en Ag NANBs sont suffisamment concentrées pour devenir réactives dans le test de DRI pour l'Ag HBs, en raison de la faible réaction croisée mentionnée ci-dessus.

La purification de l'Ag NANBs peut aussi être effectuée comme décrit par KAPLAN, Vox Sanguinis, 1978, Vol. 35, p. 224-233.

## Revendications

1. Procédé de préparation d'antigènes purifiés de l'hépatite virale NANB, caractérisé par le fait que l'on sélectionne des sérums ou des extraits de foie dans lesquels on a reconnu la présence de virus NANB, que l'on procède à une ultracentrifugation pour concentrer lesdits virus dans le culot de centrifugation, que l'on recueille ledit culot et le traite par un détergent non ionique, que l'on laisse incuber pendant 1 à 24 heures environ à température comprise entre 0 et 37 °C, que l'on procède à un fractionnement et repère les fractions contenant l'Ag NANBc purifié, selon les techniques connues, notamment les techniques basées sur la réaction antigène-anticorps, isole les fractions contenant de l'Ag NANBc purifié, puis que, si désiré, on soumet lesdites fractions à l'action d'un détergent ionique, laisse incuber pendant 1 heure à 24 heures environ à température de 0 à 37 °C, et isole l'Ag NANBe.

2. Procédé de préparation d'un antigène purifié de l'hépatite virale NANB, caractérisé par le fait que l'on sélectionne des sérums positifs pour l'Ag NANB désiré (NANBe ou NANBs), on élimine les particules virales éventuellement présentes, par exemple par ultracentrifugation, on élimine les gammaglobulines selon les méthodes connues, on concentre ledit Ag NANB, notamment par précipitation, puis on chromatographie les fractions concentrées contenant l'Ag NANB désiré sur un support sur lequel est fixé de l'héparine, on élue avec une solution aqueuse de chlorure de sodium de concentration croissante, recueille les éluats contenant l'Ag NANB désiré, et élimine les sels selon les méthodes usuelles.

3. Procédé de préparation d'antigènes purifiés de l'hépatite virale NANB, caractérisé par le fait que l'on hyperimmunise des animaux avec une fraction purifiée en un antigène de l'hépatite B, que l'on utilise les gammaglobulines produites par les animaux hyperimmunisés pour obtenir des lignes de précipitation avec des sérums contenant un antigène NANB correspondant et exempts d'antigène HB, et que l'on utilise les gammaglobulines ayant donné des lignes de précipitation pour réaliser les supports de chromatographie d'affinité qui permettront de fixer et purifier ledit antigène NANB.

4. Réactif permettant le diagnostic de l'hépatite virale NANB ou permettant de révéler le stade d'évolution de la maladie, caractérisé par le fait qu'il comprend un support solide sur lequel est fixé au moins un antigène NANBc, NANBe ou NANBs et/ou au moins un anticorps spécifique anti-NANBc, anti-NANBe ou anti-NANBs.

5. Réactif selon la revendication 4, caractérisé par le fait qu'il est constitué par un support solide sur lequel est fixé au moins un antigène NANBc, NANBe ou NANBs.

6. Réactif selon la revendication 5, caractérisé par le fait qu'il contient, fixé sur le support solide, de l'antigène NANBc.

7. Réactif selon l'une quelconque des revendications 4 à 6, caractérisé par le fait qu'il contient, fixé sur le support solide, de l'antigène NANBs.

8. Réactif selon l'une quelconque des revendicartions 4 à 7, caractérisé par le fait qu'il contient, fixé sur le support solide, de l'antigène NANBe.

9. Réactif selon la revendication 5, caractérisé par le fait que sur ledit support sont fixés des anticorps-anti-(gamma-globulines humaines) et que ledit antigène est présenté séparément et peut-être fixé par l'intermédiaire des anticorps correspondants du sérum à tester s'ils sont présents.

10. Réactif selon la revendication 9, caractérisé par le fait que les anticorps anti-(gammaglobulines humaines) sont des anticorps anti-IgM humaines.

11. Réactif selon la revendication 9, caractérisé par le fait que les anticorps anti-(gammaglobulines humaines) sont des anticorps anti-IgG humaines.

12. Réactif selon la revendication 4, caractérisé par le fait qu'il est constitué par un support solide sur lequel est fixé au moins un anticorps spécifique anti-NANBc, anti-NANBe ou anti-NANBs.

13. Réactif selon la revendication 4, caractérisé par le fait qu'il contient, fixé sur le support, de l'anticorps anti-NANBe et anti-NANBs, et de l'antigène NANBc.

14. Procédé de recherche d'antigènes NANB ou d'anticorps correspondants, caractérisé par le fait que l'on recherche selon le cas, la présence d'un ou plusieurs antigènes ou d'un ou plusieurs anticorps dans un sérum à tester, à l'aide du réactif de l'une quelconque des revendications 4 à 8 et 12, selon les techniques de « sandwich » et/ou selon les techniques de « compétition ».

15. Procédé de recherche d'anticorps anti-NANB, caractérisé par le fait qu'après mise en contact du réactif de l'une quelconque des revendications 9 à 11 avec le sérum à tester, puis avec l'antigène purifié, on le met en contact avec un anticorps anti-NANB couplé à un traceur et que l'on détermine, par une réaction de révélation, si l'anticorps couplé à un traceur s'est fixé sur le support, auquel cas les gammaglobulines du sérum à tester contenaient des anticorps contre le même antigène que ledit anticorps couplé au traceur.

16. Procédé de recherche d'antigènes NANBs et NANBe, et de recherche d'anticorps anti-NANBc, caractérisé par le fait que l'on recherche à l'aide du réactif de la revendication 13, selon la technique sandwich ou la technique de compétition, ces recherches étant effectuées l'une à l'aide d'un traceur radioactif, l'autre à l'aide d'un traceur enzymatique.

**Claims**

1. Process for the preparation of purified antigens of viral hepatitis NANB, characterized by the selection of serums or liver extracts wherein the presence of NANB virus has been recognized, ultracentrifugation to concentrate said viruses in the centrifugation base, the collection and treatment of said base by means of a non-ionic detergent, allowing to incubate during 1 to 24 hours approximately at a temperature comprised between 0 and 37 °C, fractionation and detection of the fractions containing the purified Ag NANBc using known techniques, especially techniques which are based on the antigen-antibody reaction, isolating the fraction containing the purified Ag NANBc and then, if desired, subjecting said fractions to the action of an ionic detergent, incubating during 1 to 24 hours approximately at a temperature of 0 to 37 °C, and isolating the Ag NANBe.

2. Process for the preparation of a purified antigen of viral hepatitis NANB, characterized by the selection of serums which are positive to the desired Ag NANB (NANBe or NANBs), the removal of the virus particles which may be present, e. g., by ultra centrifugation, the removal of the gammaglobulins by known methods, the concentration of said Ag NANB, particularly by precipitation, then the chromatography of the concentrated fractions which contain the desired Ag NANB on a support on which heparin is fixed, elution with an aqueous solution of sodium chloride in increasing concentration, collection of the eluates which contain the desired Ag NANB, and removal of the salts by known methods.

3. Process for the preparation of purified antigens of viral hepatitis NANB, characterized by the hyperimmunization of animals with a purified fraction of a hepatitis B antigen, the use of the gammaglobulins produced by the hyperimmunized animals to obtain lines of precipitation with serums containing a corresponding NANB antigen and without HB antigen, and the use of the gammaglobulins which have formed precipitation lines to create supports for affinity chromatography which will make it possible to fix and purify said NANB antigen.

4. Reagent which makes it possible to diagnose viral hepatitis NANB or makes it possible to detect the stage of development of the disease, characterized by comprising a solid support on which is fixed at least one NANBc, NANBe, or NANBs antigen and/or at least one anti-NANBc, anti-NANBe, or anti-NANBs specific antibody.

5. Reagent according to claim 4, characterized by consisting of a solid support on which is fixed at least one NANBc, NANBe, or NANBs antigen.

6. Reagent according to claim 5, characterized by containing NANBc antigen fixed on the solid support.

7. Reagent according to any one of claims 4 to 6, characterized by containing NANBs antigen fixed on the solid support.

8. Reagent according to any one of claims 4 to 7, characterized by containing NANBe antigen fixed on the solid support.

9. Reagent according to claim 5, characterized by fixation of anti- (human gammaglobulin) antibodies on said support and said antigen being present separately and the ability of said antigen to be fixed by corresponding antibodies of the serum to be tested, if they are present.

10. Reagent according to claim 9, characterized by the fact that the anti- (human gammaglobulin) antibodies are anti-human IgM antibodies.

11. Reagent according to claim 9, characterized by the fact that the anti- (human gammaglobulin) antibodies are anti-human IgG antibodies.

12. Reagent according to claim 4, characterized by consisting of a solid support on which is fixed at least one anti-NANBc, anti-NANBe, or anti-NANBs specific antibody.

13. Reagent according to claim 4, characterized by the fact that it contains, fixed on the support, anti-NANBe and anti-NANBs antibodies and NANBc antigen.

14. Process for detection of NANB antigens or of corresponding antibodies, characterized by, according to the case, the searching for the presence of one or more antigens or one more antibodies in a serum to be tested, using the reagent of any one of claims 4 to 8 and 12, according to the « sandwich » and/or « competition » techniques.

15. Process for detection of anti-NANB antibodies, characterized by contacting the reagent of any one of claims 9 to 11, after it was contracted with the serum to be tested and then with the purified antigen, with an anti-NANB antibody coupled with a tracer and by determining by means of a detection reaction whether the antibody coupled with the tracer has become fixed on the support, in which case the gammaglobulins of the serum to be tested contain antibodies against the same antigen as the antibody coupled with the tracer.

16. Process for detection of NANBs and NANBe antigens and of anti-NANBc antibodies, characterized by using the reagent of claim 13, according to the sandwich technique or the competition technique, with these searches being carried out, in the first case, using a radioactive tracer and, in the second case, using an enzyme tracer.

**Patentansprüche**

1. Verfahren zur Herstellung gereinigter Antigene der NANB-Virushepatitis, dadurch gekennzeichnet, daß man Sera oder Leberextrakte wählt, in denen man die Anwesenheit von NANB-Viren festgestellt hat, daß man ultrazentrifugiert, um die Viren im Zentrifugenrückstand zu konzentrieren, daß man diesen Rückstand gewinnt und mit einem nichtionischen Detergens behandelt, daß man etwa 1 bis 24 Stunden bei einer Temperatur von 0 bis 37 °C inkubiert, daß man fraktioniert und festellt, welche Fraktionen das gereinigte NANBc-Ag enthalten, wobei man gemäß bekannten Techniken verfährt, insbesondere den Techniken, die auf der Antigen-Antikörperreaktion basieren, die das gereinigte NANBc-Ag enthaltenden Fraktionen isoliert, dann, falls gewünscht, diese Fraktionen der Einwirkung eines ionischen Detergens unterwirft, etwa 1 bis 24 Stunden bei einer Temperatur von 0 bis 37 °C inkubieren läßt, und das NANBe-Ag isoliert.

2. Verfahren zur Herstellung eines gereinigten Antigens der NANB-Virushepatitis, dadurch gekennzeichnet, daß man Sera wählt, die positiv sind auf das gewünschte NANB-Ag (NANBe oder NANBs), gegebenenfalls vorhandene virale Partikel entfernt, beispielsweise durch Ultrazentrifugieren, die $\gamma$-Globuline nach bekannten Verfahren elminiert, das NANB-Ag konzentriert, insbesondere durch Ausfällen, dann die das gewünschte NANB-Ag enthaltenden konzentrierten Fraktionen an einem Träger, an dem Heparin fixiert ist, chromatographiert, mit einer wäßrigen Natriumchloridlösung mit zunehmender Konzentration eluiert, die das gewünschte NANB-Ag enthaltenden Eluate gewinnt und die Salze nach üblichen Verfahren eliminiert.

3. Verfahren zur Herstellung gereinigter Antigene der NANB-Virushepatitis, dadurch gekennzeichnet, daß man Tiere mit einer gereinigten Fraktion an einem Hepatitis-B-Antigen hyperimmunisiert, daß man die durch die hyperimmunisierten Tiere erzeugten $\gamma$-Globuline einsetzt, um mit den Sera, die ein entsprechendes NANB-Antigen enthalten und die frei von HB-Antigen sind, Präzipitationslinien zu erhalten, und daß man die $\gamma$-Globuline, welche zu den Präzipitationslinien geführt haben, zur Herstellung der Träger für die Affinitätschromatographie verwendet, welche die Fixierung und Reinigung des NANB-Antigens ermöglichen.

4. Reagenz, mit dem die NANB-Virushepatitis diagnostiziert werden kann oder das es ermöglicht, das Entwicklungsstadium der Krankheit festzustellen, dadurch gekennzeichnet, daß es einen festen Träger aufweist, auf dem mindestens ein NANBc-, NANBe- oder NANBs-Antigen und/oder mindestens ein Anti-NANBc-, Anti-NANBe- oder Anti-NANBs-spezifischer-Antikörper fixiert ist.

5. Reagenz nach Anspruch 4, dadurch gekennzeichnet, daß es aus einem festen Träger besteht, auf dem mindestens ein NANBc-, NANBe- oder NANBs-Antigen fixiert ist.

6. Reagenz nach Anspruch 5, dadurch gekennzeichnet, daß es NANBc-Antigen enthält, das auf den festen Träger fixiert ist.

7. Reagenz nach einem der Ansprüche 4 bis 6, dadurch gekennzeichnet, daß es NANBs-Antigen enthält, das auf dem festen Träger fixiert ist.

8. Reagenz nach einem der Ansprüche 4 bis 7, dadurch gekennzeichnet, daß es NANBe-Antigen enthält, das auf dem festen Träger fixiert ist.

9. Reagenz nach Anspruch 5, dadurch gekennzeichnet, daß auf dem Träger Anti-(human-Gammaglobuline)-Antikörper fixiert sind und daß das Antigen abgetrennt davon vorhanden ist und mittels der entsprechenden Antikörper des zu testenden Serums fixiert werden kann, falls diese vorhanden sind.

10. Reagenz nach Anspruch 9, dadurch gekennzeichnet, daß die Anti-(human-Gammaglobuline)-Antikörper Anti-human-Igm-Antikörper sind.

11. Reagenz nach Anspruch 9, dadurch gekennzeichnet, daß die Anti-(human-Gammaglobuline)-Antikörper Anti-human-IgG-Antikörper sind.

12. Reagenz nach Anspruch 4, dadurch gekennzeichnet, daß es aus einem festen Träger besteht, auf dem mindestens ein Anti-NANBc-, Anti-NANBe- oder Anti-NANBs-spezifischer-Antikörper fixiert ist.

13. Reagenz nach Anspruch 4, dadurch gekennzeichnet, daß es auf dem Träger fixiert Anti-NANBe- und Anti-NANBs-Antikörper und NANBc-Antigen enthält.

14. Verfahren zum Auffinden von NANB-Antigenen oder entsprechenden Antikörpern, dadurch gekennzeichnet, daß man mit Hilfe des Reagenz nach einem der Ansprüche 4 bis 8 und 12 gemäß den « sandwich »- und/oder « kompetitiven » Techniken jeweils feststellt, ob ein oder mehrere Antigene oder ein oder mehrere Antikörper im Testserum vorhanden ist (sind).

15. Verfahren zum Auffinden von Anti-NANB-Antikörpern, dadurch gekennzeichnet, daß man das Reagenz nach einem der Ansprüche 9 bis 11, nachdem man es mit dem Testserum und dann mit dem gereinigten Antigen in Kontakt gebracht hat, mit einem an einen Tracer gekoppelten Anti-NANB-Antikörper in Kontakt bringt und daß man mittels einer Entwicklungsreaktion feststellt, ob der an einen Tracer gekoppelt Antikörper an dem Träger fixiert ist, wobei in diesem Fall die $\gamma$-Globuline des Testserums Antikörper gegen dasselbe Antigen wie der an den Tracer gekoppelte Antikörper enthalten.

16. Verfahren zum Auffinden von NANBs- und NANBe-Antigenen und zum Auffinden von Anti-NANBc-Antikörpern, dadurch gekennzeichnet, daß man das Verfahren mit Hilfe des Reagenz nach Anspruch 13 gemäß der Sandwich-Tecknik oder gemäß der kompetitiven Technik durchführt, wobei man einmal einen radioaktiven Tracer und das andere Mal einen enzymatischen Tracer einsetzt.